# EUROPEAN PATENT SPECIFICATION

(11) **EP 3 676 404 B1**
(45) Date of publication and mention of the grant of the patent: **03.09.2025**
(21) Application number: 18782185.5
(22) Date of filing: 21.08.2018
(51) Int. Cl.: C12Q 1/6886

(54) **METHODS OF SELECTING A TREATMENT FOR BREAST CANCER PATIENTS**
VERFAHREN ZUR AUSWAHL EINER BEHANDLUNG FÜR KREBSPATIENTEN
PROCÉDÉS DE SÉLECTION D'UN TRAITEMENT POUR DES PATIENTS ATTEINTS D'UN CANCER

(30) Priority: 31.08.2017 US 201762552842 P
(43) Date of publication of application: 08.07.2020
(73) Proprietor: Novartis AG, 4056 Basel (CH)
(72) Inventor: SU, Fei, East Hanover, New Jersey 07936 (US); GERMA, Marie-Caroline, East Hanover, New Jersey 07936 (US); MILLER, Michelle, East Hanover, New Jersey 07936 (US); HE, Wei, Cambridge, Massachusetts 02139 (US)
(74) Representative: Carpmaels & Ransford LLP
(86) International application number: PCT/IB2018/056318
(87) International publication number: WO 2019/043504

(56) References cited:
- EP-A1- 1 788 083
- WO-A1-2015/134674
- ANDRE FABRICE ET AL: "Abstract CT045: Ribociclib + letrozole for first-line treatment of hormone receptor-positive (HR+), human epidermal growth factor receptor 2-negative (HER2-) advanced breast cancer (ABC): efficacy by baseline tumor markers | Cancer Research | American Association for Cancer Research", CANCER RES, vol. 77, no. 13S, 1 July 2017 (2017-07-01), XP093002172, Retrieved from the Internet <URL:https://aacrjournals.org/cancerres/article/77/13_Supplement/CT045/622263/Abstract-CT045-Ribociclib-letrozole-for-first-line> DOI: :10.1158/1538-7445.AM2017-CT045
- G. E. KONECNY ET AL: "Expression of p16 and Retinoblastoma Determines Response to CDK4/6 Inhibition in Ovarian Cancer", CLINICAL CANCER RESEARCH, vol. 17, no. 6, 28 January 2011 (2011-01-28), US, pages 1591 - 1602, XP055223034, ISSN: 1078-0432, DOI: 10.1158/1078-0432.CCR-10-2307
- RICHARD S FINN ET AL: "The cyclin-dependent kinase 4/6 inhibitor palbociclib in combination with letrozole versus letrozole alone as first-line treatment of oestrogen receptor-positive, HER2-negative, advanced breast cancer (PALOMA-1/TRIO-18): a randomised phase 2 study", THE LANCET. ONCOLOGY, 1 January 2015 (2015-01-01), England, pages 25 - 35, XP055375445, Retrieved from the Internet <URL:http://www.thelancet.com/pdfs/journals/lanonc/PIIS1470-2045(14)71159-3.pdf> DOI: 10.1016/S1470-2045(14)71159-3
- RICHARD S. FINN ET AL: "Palbociclib and Letrozole in Advanced Breast Cancer", THE NEW ENGLAND JOURNAL OF MEDICINE, - NEJM -, vol. 375, no. 20, 17 November 2016 (2016-11-17), US, pages 1925 - 1936, XP055375437, ISSN: 0028-4793, DOI: 10.1056/NEJMoa1607303
- R FINN ET AL: "Biomarker analyses from the phase 3 PALOMA-2 trial of palbociclib (P) with letrozole (L) compared with placebo (PLB) plus L in postmenopausal women with ER + /HER2- advanced breast cancer (ABC)", ANNALS OF ONCOLOGY, 13 October 2016 (2016-10-13), XP055520627, Retrieved from the Internet <URL:https://academic.oup.com/annonc/article/27/suppl_6/LBA15/2800514> [retrieved on 20181031], DOI: 10.1093/annonc/mdw435.5

## Description

### TECHNICAL FIELD

The disclosure is directed to predictive methods, personalized therapies, kits, transmittable forms of information and methods for treating patients having cancer.

### BACKGROUND OF THE DISCLOSURE

Breast cancer is a heterogeneous disease with a high degree of diversity in histology, therapeutic response, and patient treatment outcomes. Each year 1.1 million new cases of breast cancer occur among women worldwide and 400,000 women will die from this disease. Although progress has been made in preventing deaths by breast cancer in women, we still do not understand the biology of breast cancer at a level that would explain why certain patients respond well to therapy, whereas others develop recurrent disease with an inexorable downhill course. Improved treatment options are urgently needed to end this stalemate and to treat breast cancer patients effectively.

WO2015/134674 A1 relates to biomarkers indicating sensitivity or resistance to Cyclin D - CDK4/6 inhibitor therapy in cancer.

Andre Fabrice ET AL, Cancer Res, vol. 77, no. 13S, 1 July 2017 relates to the Phase III MONALEESA-2 study of ribociclib + letrozole vs. placebo + letrozole for first-line treatment of HR+, HER2- advanced breast cancer, assessed in baseline tumors by protein levels of Rb, p16, the cell proliferation marker Ki67, and by gene expression levels of CDKN2A (p16) and CCND1.

### BRIEF SUMMARY OF THE DISCLOSURE

The present finding is based on the novel predictive methods and personalized therapies for patients having cancer, e.g., breast cancer, that identify a patients prognosis and can be used to select patients most likely to benefit from treatment with a combination of a CDK 4/6 inhibitor and an aromatase inhibitor. Specifically, the present invention is based on the finding that using the biomarker(s) of the invention it is possible to predict which breast cancer patients show an increased chance of progression free survival (PFS) if treated with a combination of ribociclib and letrozole compared with patients treated with letrozole alone. The specific invention is defined in the appended claims.

In one embodiment, the invention includes a CDK 4/6 inhibitor which is ribociclib, or a pharmaceutically acceptable salt, solvate, hydrate or cocrystal thereof, for use in a method of selectively treating a patient having breast cancer, comprising: a) assaying a biological sample from the patient for the level of expression of one or more of CCNA2, CCNE1, CDK2, CDK4, CDKN1A, CDKN2C, E2F1, E2F3, and RB1; and thereafter, selectively administering a therapeutically effective amount of the CDK 4/6 inhibitor and an aromatase inhibitor selected from the group consisting of letrozole, anastrozole and exemestane to the patient if the patient has an increased level of expression or one or more of CCNA2, CCNE1, CDK2, CDK4, CDKN1A, CDKN2C, E2F1, E2F3, and RB1 relative to a control.

In another aspect, the invention includes a method of predicting the likelihood that a patient having cancer will respond to treatment with a CDK 4/6 inhibitor which is ribociclib, or a pharmaceutically acceptable salt, solvate, hydrate or cocrystal thereof, and an aromatase inhibitor, selected from the group consisting of letrozole, anastrozole and exemestane, including assaying a biological sample from the patient for the level of expression of one or more of CCNA2, CCNE1, CDK2, CDK4, CDKN1A, CDKN2C, E2F1, E2F3, and RB1, wherein an increase in the level of expression of one or more of CCNA2, CCNE1, CDK2, CDK4, CDKN1A, CDKN2C, E2F1, E2F3, and RB1 relative to a control is indicative of an increased likelihood that the patient will respond to treatment with the CDK 4/6 inhibitor and the aromatase inhibitor.

In yet another aspect, the invention includes a CDK 4/6 inhibitor, which is ribociclib or a pharmaceutically acceptable salt, solvate, hydrate or cocrystal thereof, and an aromatase inhibitor selected from the group consisting of letrozole, anastrozole and exemestane for use in a method of treating a patient having breast cancer including:
a) selecting the patient for treatment with a CDK 4/6 inhibitor and an aromatase inhibitor on the basis of the patient having an increase in the level of expression of one or more of CCNA2, CCNE1, CDK2, CDK4, CDKN1A, CDKN2C, E2F1, E2F3, and RB1 relative to a control; and
b) thereafter, administering a therapeutically effective amount of the CDK 4/6 inhibitor and the aromatase inhibitor to the patient.

In still yet another aspect, the invention further includes an initial step of assaying a biological sample from the patient for the level of expression of one or more of CCNA2, CCNE1, CDK2, CDK4, CDKN1A, CDKN2C, E2F1, E2F3, and RB1.

In still yet another aspect, the absence of an increase in the level of expression of one or more of CCNA2, CCNE1, CDK2, CDK4, CDKN1A, CDKN2C, E2F1, E2F3, and RB1 is indicative of a decreased likelihood that the patient will respond to treatment with the CDK 4/6 inhibitor and the aromatase inhibitor.

In a final aspect, the invention includes ribociclib in combination with letrozole for use in a method of treating a postmenopausal female patient with HR-positive, HER2- negative advanced breast cancer, who has previously received no prior therapy for advanced breast cancer, comprising: assaying a biological sample from the patient for the level of expression of one or more of CCNA2, CCNE1, CDK2, CDK4, CDKN1A, CDKN2C, E2F1, E2F3, and RB1; and thereafter, selectively administering a therapeutically effective amount of ribociclib and letrozole to the patient if the patient has an increased level of expression or one or more of CCNA2, CCNE1, CDK2, CDK4, CDKN1A, CDKN2C, E2F1, E2F3, and RB1 relative to a control.

The biological sample used in the methods above can be any tumor sample.

In the methods of the above, the step of assaying the level of expression of one or more genes can be performed by detecting gene expression, e.g., mRNA expression. Techniques selected from the group consisting of Northern blot analysis, polymerase chain reaction (PCR), reverse transcription-polymerase chain reaction (RT-PCR), or TaqMan-based assays can be used.

Another aspect of the present disclosure not part of the invention includes a method for the prognosis of a cancer patient comprising: measuring in a biological sample obtained from said subject the gene expression level of at least one or more genes selected from the group consisting of CCNA2, CCNE1, CDK2, CDK4, CDKN1A, CDKN2B, CDKN2C, E2F1, E2F3, and RB1, b) comparing every expression level determined at step a) with a control, wherein comparison of said expression level to said control is predictive of the prognosis of cancer in the subject. In one embodiment, if the expression of the gene measured in a) is higher than the control than the subject is determined to have a poor prognosis, e.g., has a decreased chance of progression free survival (PFS). In another embodiment, if the expression of the gene measured in a) is at or lower than the control than the subject is determined to have a good prognosis, e.g., has an increased chance of progression free survival (PFS).

In one embodiment of the invention, according to the methods above, the CDK 4/6 inhibitor, ribociclib, and an aromatase inhibitor can be administered either a fixed combination in one dosage unit form or a kit of parts for the combined administration where the CDK 4/6 inhibitor and the aromatase inhibitor may be administered independently at the same time or separately within time intervals that allow that the therapeutic agents to have a therapeutic effect.

The term "comprising" encompasses "including" as well as "consisting," e.g. a composition "comprising" X may consist exclusively of X or may include something additional, e.g., X + Y.

The term "about" in relation to a numerical value x means +/-10% unless the cotext dictates otherwise.

As used herein, the terms "subject" and "patient" include any human or nonhuman animal. The term "nonhuman animal" includes all vertebrates, e.g., mammals and non-mammals, such as nonhuman primates, sheep, dogs, cats, horses, cows, chickens, amphibians, reptiles, etc.

The term "assaying" is used to refer to the act of identifying, screening, probing, testing measuring or determining, which act may be performed by any conventional means. For example, a sample may be assayed for the presence of a particular genetic marker by using an a Northern blot. The term "detecting" (and the like) means the act of extracting particular information from a given source, which may be direct or indirect. In some embodiments of the predictive methods disclosed herein, the presence of a given thing (e.g., level of gene expression, etc.) is detected in a biological sample. The terms "assaying" and "determining" contemplate a transformation of matter, e.g., a transformation of a biological sample, e.g., a tumor sample, from one state to another by means of subjecting that sample to physical testing.

The term "obtaining" means to procure, e.g., to acquire possession of in any way, e.g., by physical intervention (e.g., biopsy) or non-physical intervention (e.g, transmittal of information via a server), etc.

The term "combination", as used herein, defines either a fixed combination in one dosage unit form or a kit of parts for the combined administration where the CDK 4/6 inhibitor and the aromatase inhibitor may be administered independently at the same time or separately within time intervals that allow that the therapeutic agents.

The term "pharmaceutical composition" is defined herein to refer to a mixture or solution containing at least one therapeutic agent to be administered to a subject, e.g., a mammal or human, in order to prevent or treat a particular disease or condition affecting the mammal.

The term "pharmaceutically acceptable" is defined herein to refer to those compounds, materials, compositions and/or dosage forms, which are, within the scope of sound medical judgment, suitable for contact with the tissues a subject, e.g., a mammal or human, without excessive toxicity, irritation allergic response and other problem complications commensurate with a reasonable benefit/risk ratio.

The term "a combined preparation" is defined herein to refer to especially a "kit of parts" in the sense that the therapeutic agents (a) and (b), for example, the CDK 4/6 inhibitor and the aromatase inhibitor, can be dosed independently or by use of different fixed combinations with distinguished amounts of the therapeutic agents (a) and (b), i.e., simultaneously or at different time points. The parts of the kit of parts can then e.g., be administered simultaneously or chronologically staggered, that is at different time points and with equal or different time intervals for any part of the kit of parts. The ratio of the total amounts of the therapeutic agent (a) to the therapeutic agent (b) to be administered in the combined preparation can be varied, e.g., in order to cope with the needs of a patient sub-population to be treated or the needs of the single patient.

The term "combined administration" or "administration of the combination" as used herein is defined to encompass the administration of the selected therapeutic agents to a single patient, and are intended to include treatment regimens in which the agents are not necessarily administered by the same route of administration or at the same time.

The term "treating" or "treatment" as used herein comprises a treatment relieving, reducing or alleviating at least one symptom in a subject or effecting a delay of progression of a cancer, e.g., breast cancer. For example, treatment can be the diminishment of one or several symptoms of a cancer or complete eradication of a cancer. Within the meaning of the present invention, the term "treat" also denotes to arrest, delay the onset (i.e., the period prior to clinical manifestation of a cancer of the head and neck) and/or reduce the risk of developing or worsening a cancer of the head and neck. The term "prevention" is used herein to mean prevent, delay or treat, or all, as appropriate, development or continuance or aggravation of a cancer of the head and neck in a subject.

The term "administering" in relation to a compound, e.g., CDK 4/6 or another agent, is used to refer to delivery of that compound to a patient by any route.

As used herein, a "therapeutically effective amount" refers to an amount of CDK 4/6 alone or in combination with an aromatase inhibitor is effective, upon single or multiple dose administration to a patient (such as a human) for treating, preventing, preventing the onset of, curing, delaying, reducing the severity of, ameliorating at least one symptom of a disorder or recurring disorder, or prolonging the survival of the patient beyond that expected in the absence of such treatment. When applied to an individual active ingredient administered alone, the term refers to that ingredient alone. When applied to a combination, the term refers to combined amounts of the active ingredients that result in the therapeutic effect, whether administered in combination, serially or simultaneously.

The phrase "receiving data" is used to mean obtaining possession of information by any available means, e.g., orally, electronically (e.g., by electronic mail, encoded on diskette or other media), written, etc.

As used herein, "selecting" and "selected" in reference to a patient is used to mean that a particular patient is specifically chosen from a larger group of patients on the basis of (due to) the particular patient having a predetermined criteria, e.g., the patient has an increase in the expression of one or more particular genes. Similarly, "selectively treating" refers to providing treatment to a patient having cancer, where that patient is specifically chosen from a larger group of cancer patients on the basis of the particular patient having a predetermined criteria, e.g., an increase in the expression level of one or more genes. Similarly, "selectively administering" refers to administering a drug to a patient that is specifically chosen from a larger group of patients on the basis of (due to) the particular patient having a predetermined criteria, e.g., a particular genetic or other biological marker. By selecting, selectively treating and selectively administering, it is meant that a patient is delivered a personalized therapy based on the patient's particular biology, rather than being delivered a standard treatment regimen based solely on the patient having a particular disease. Selecting, in reference to a method of treatment as used herein, does not refer to fortuitous treatment of a patient that has an increase in the expression of one or more particular genes, but rather refers to the deliberate choice to administer a CDK4/6 inhibitor together with an aromatase inhibitor to a patient based on the patient having cancer such as breast cancer. Thus, selective treatment differs from standard treatment, which delivers a particular drug to all patients, regardless of their allelic status.

As used herein, "predicting" indicates that the methods described herein provide information to enable a health care provider to determine the likelihood that an individual having an increase in the expression of one or more particular genes will respond to or will respond more favorably to treatment to the combination of a CDK4/6 inhibitor and an aromatase inhibitor. It does not refer to the ability to predict response with 100% accuracy. Instead, the skilled artisan will understand that it refers to an increased probability.

As used herein, "likelihood" and "likely" is a measurement of how probable an event is to occur. It may be used interchangably with "probability". Likelihood refers to a probability that is more than speculation, but less than certainty. Thus, an event is likely if a reasonable person using common sense, training or experience concludes that, given the circumstances, an event is probable.

The phrase "increased likelihood" refers to an increase in the probability that an event will occur. For example, some methods herein allow prediction of whether a patient will display an increased likelihood of responding to treatment with a CDK4/6 inhibitor and an aromatase inhibitor or an increased likelihood of responding better to treatment with CDK4/6 inhibitor and an aromatase inhibitor in comparison to a patient having an cancer who does not have an increase in the expression of one or more genes.

The term "probe" refers to any composition of matter that is useful for specifically detecting another substance. The probe can be a PCR primer, e.g., together with another primer, for amplifying a particular gene sequence region.

The phrase "specifically hybridizes" is used to refer to hybrization under stringent hybridization conditions. Stringent conditions are known to those skilled in the art and can be found in Current Protocols in Molecular Biology, John Wiley & Sons, N.Y. (1989), 6.3.1-6.3.6. Aqueous and nonaqueous methods are described in that reference and either can be used. One example of stringent hybridization conditions is hybridization in 6X sodium chloride/sodium citrate (SSC) at about 45°C, followed by at least one wash in 0.2X SSC, 0.1% SDS at 50°C. A second example of stringent hybridization conditions is hybridization in 6X SSC at about 45°C, followed by at least one wash in 0.2X SSC, 0.1% SDS at 55°C. Another example of stringent hybridization conditions is hybridization in 6X SSC at about 45°C, followed by at least one wash in 0.2X SSC, 0.1% SDS at 60°C. A further example of stringent hybridization conditions is hybridization in 6X SSC at about 45°C, followed by at least one wash in 0.2X SSC, 0.1% SDS at 65°C. High stringent conditions include hybridization in 0.5 M sodium phosphate, 7% SDS at 65°C, followed by at least one wash at 0.2X SSC, 1% SDS at 65°C.

The phrase "a region of a nucleic acid" is used to indicate a smaller sequence within a larger sequence of nucleic acids. For example, a gene is a region of a chromosome, an exon is a region of a gene, etc.

The term "capable" is used to mean that ability to achieve a given result, e.g., a probe that is capable of detecting the presence of a particular substance means that the probe may be used to detect the particular substance.

An "oliogonucelotide" refers to a short sequence of nucleotides, e.g., 2-100 bases.

The term "biological sample" as used herein refers to a sample from a patient, which may be used for the purpose of identification, diagnosis, prediction, or monitoring.

The term "good prognosis" as used herein refers to a patient where the patient has at least one of a decrease in recurrence risk, has an increased chance of progression free survival (PFS), an increase in the likelihood of survival, an increase in the time of survival, or a decrease in the risk of metastasis.

The term "bad prognosis" as used herein refers to a patient where the patient has at least one of an increase in recurrence risk, has a decreased chance of progression free survival (PFS), a decrease in the likelihood of survival, a decrease in the time of survival, or an increase in the risk of metastasis

Additional methods, uses, and kits are provided in the the following description and appended claims. Further features, advantages and aspects of the present disclosure will become apparent to those skilled in the art from the following description and appended claims.

### BRIEF DESCRIPTION OF THE DRAWINGS

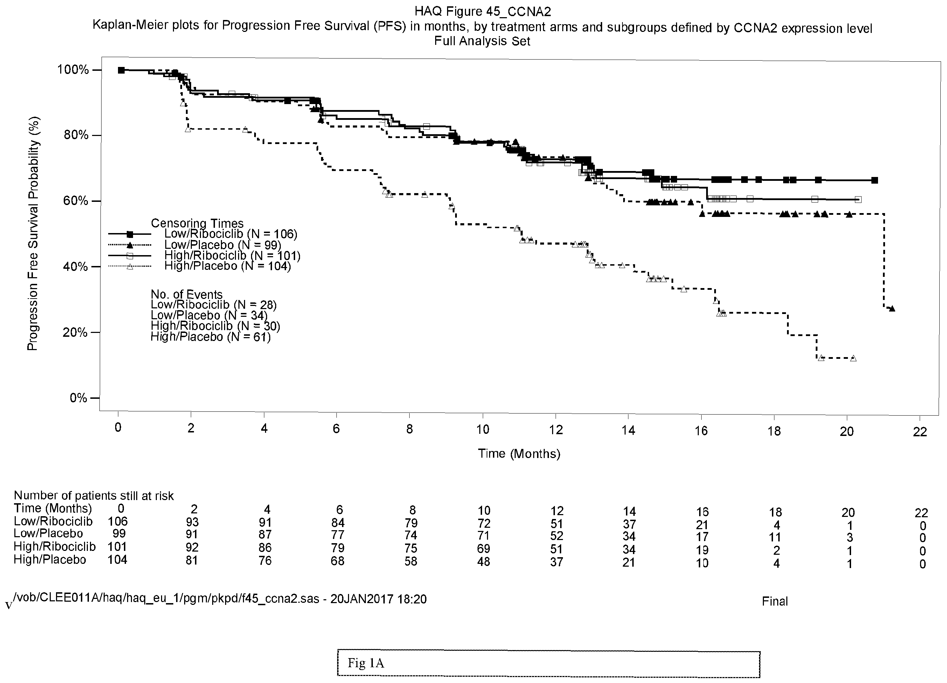

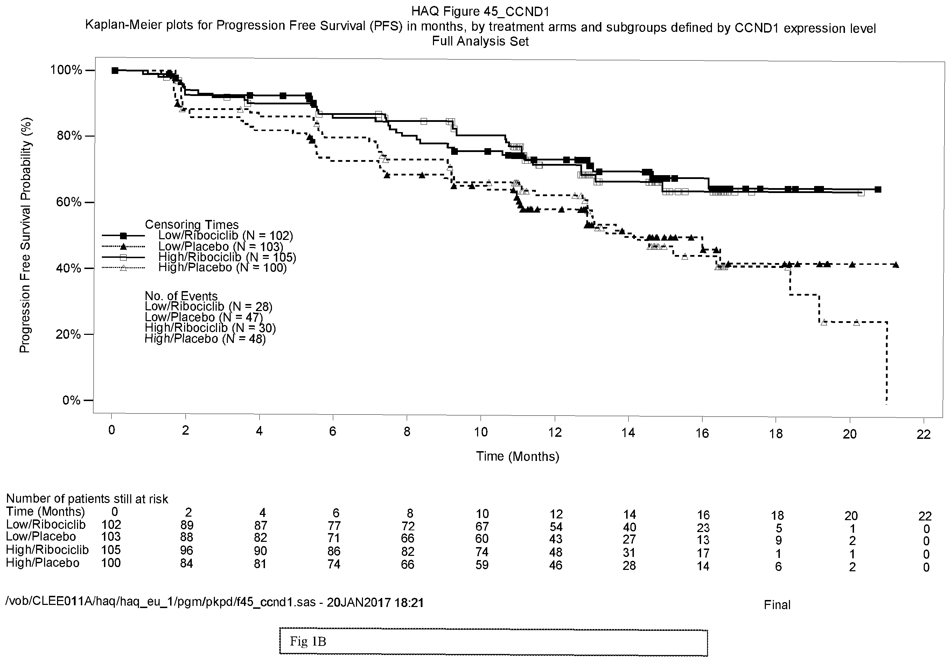

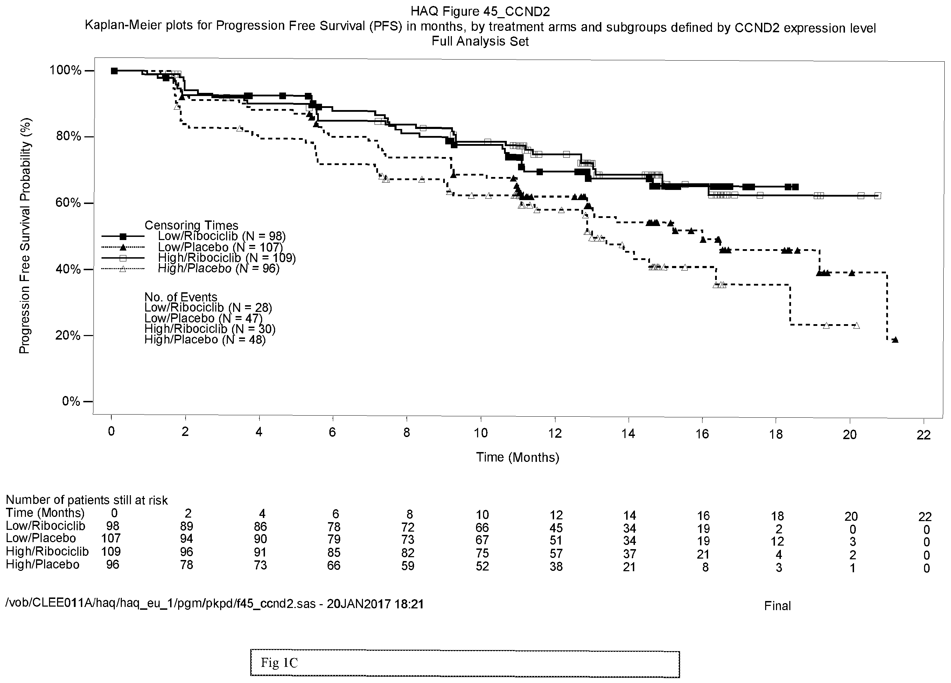

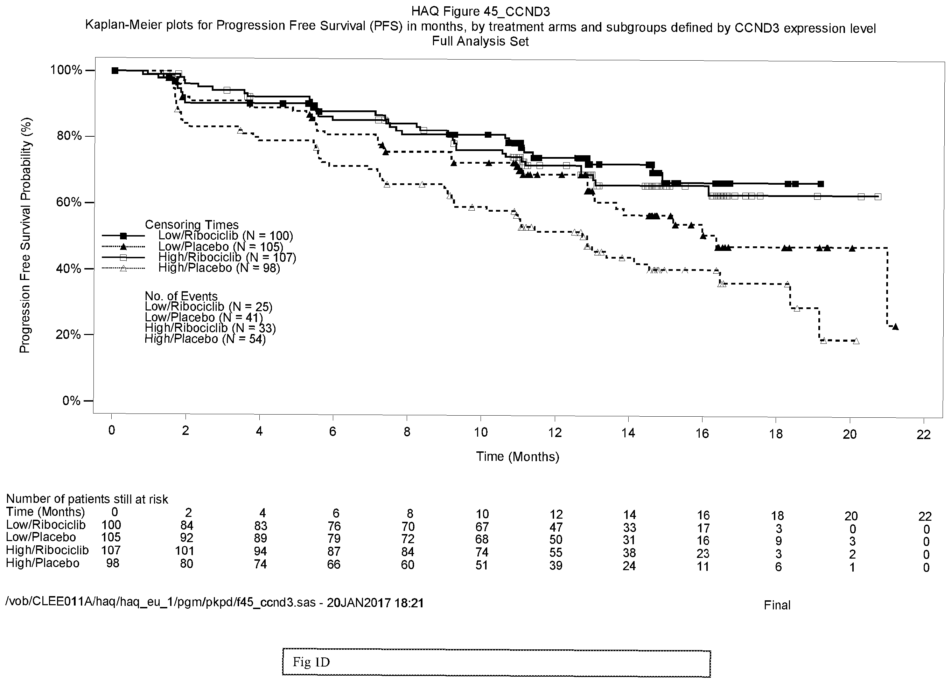

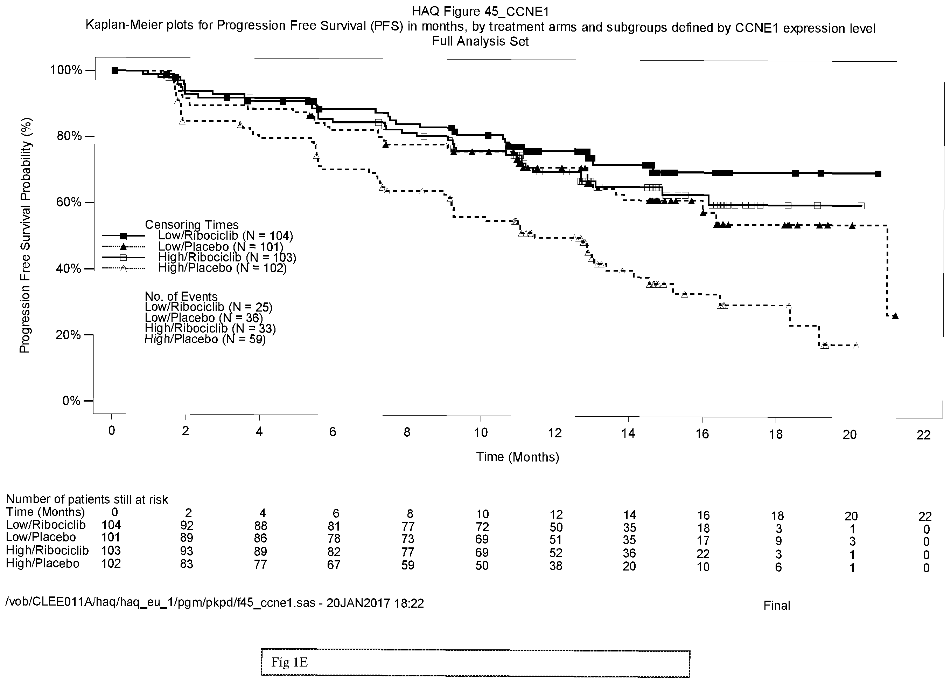

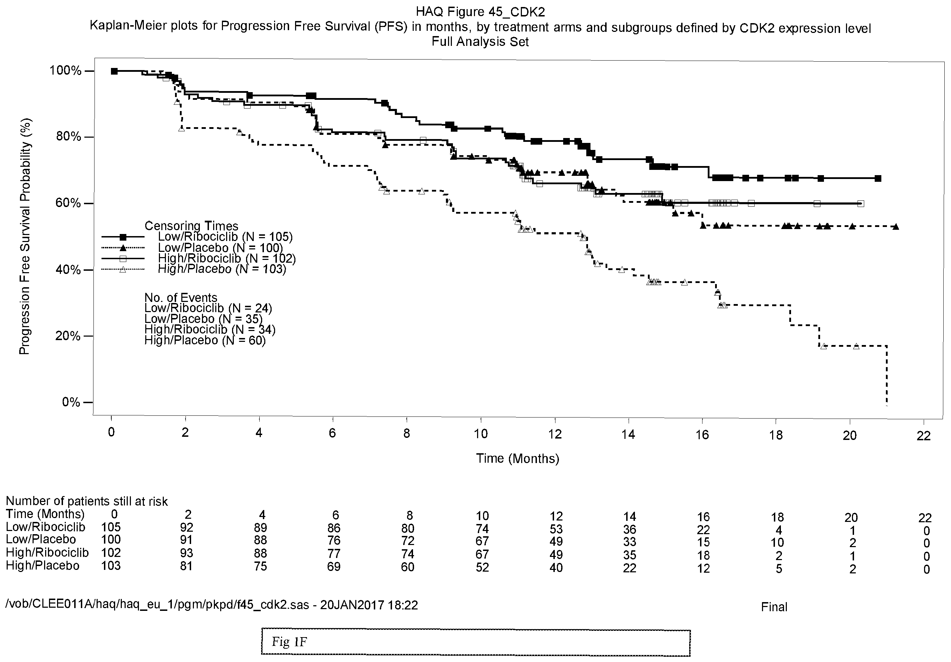

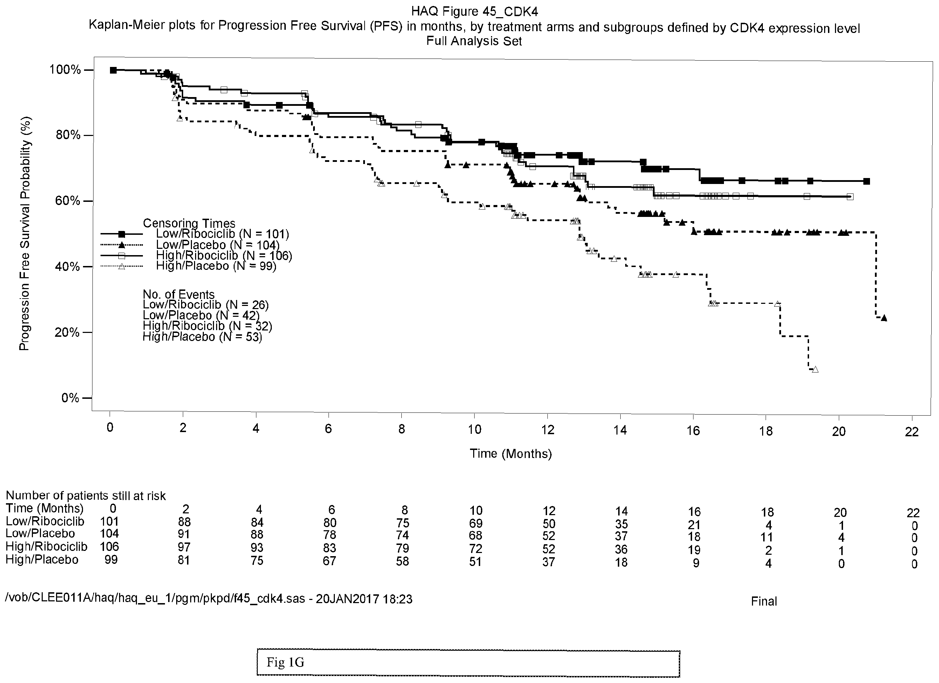

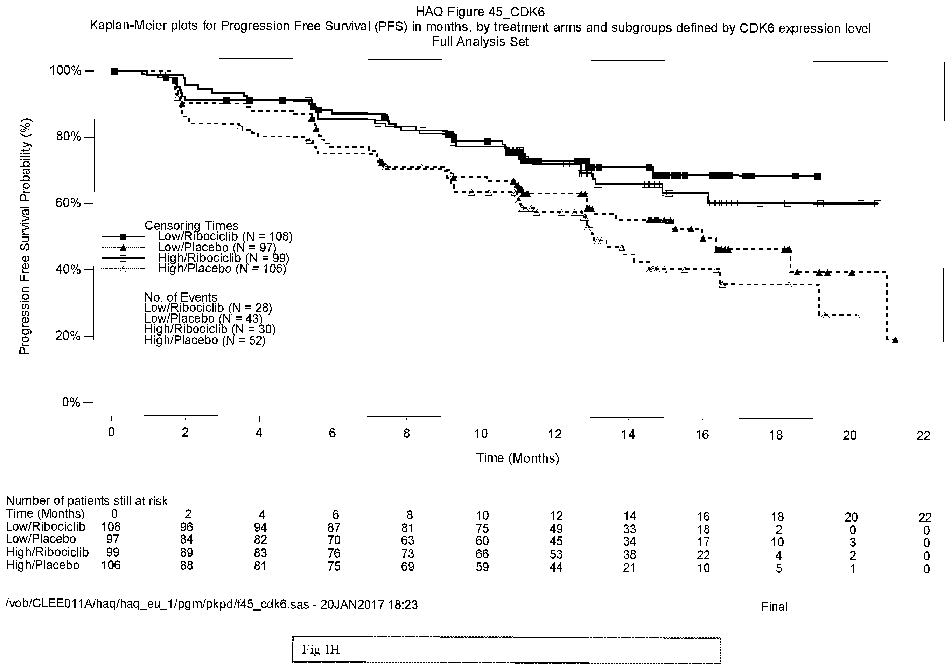

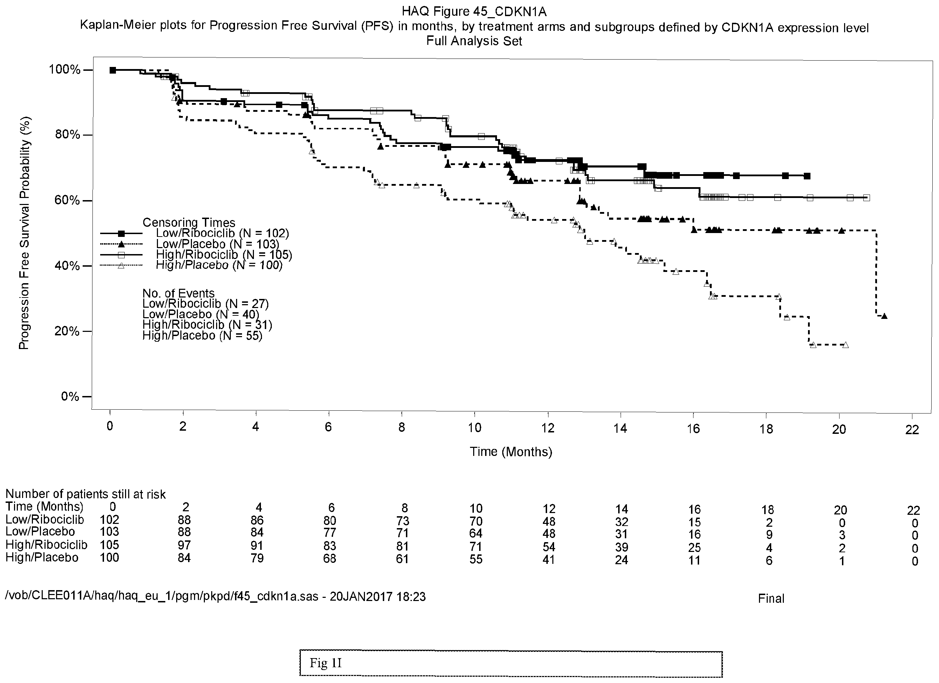

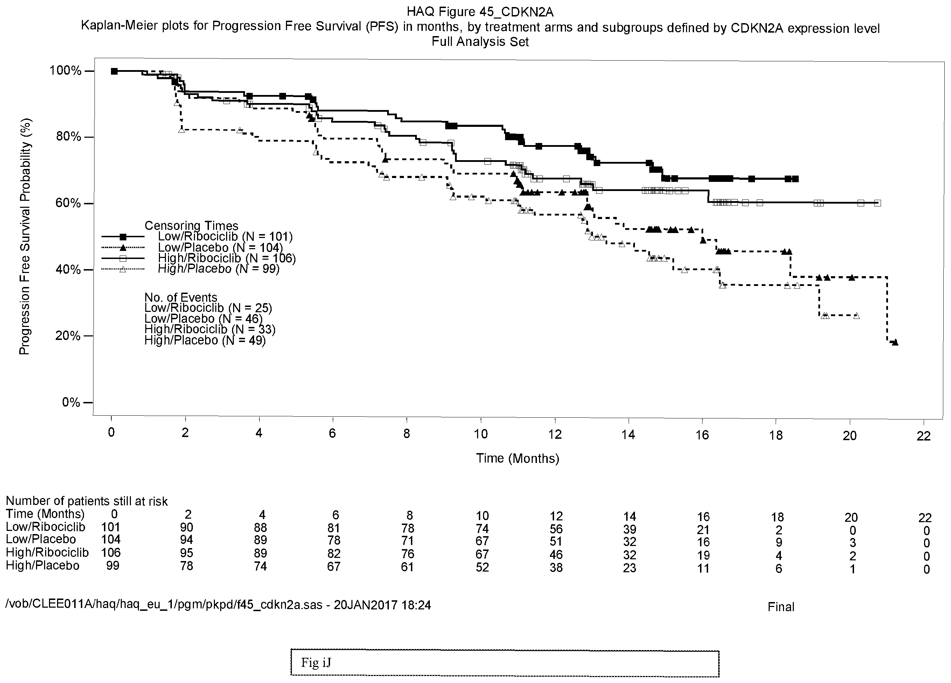

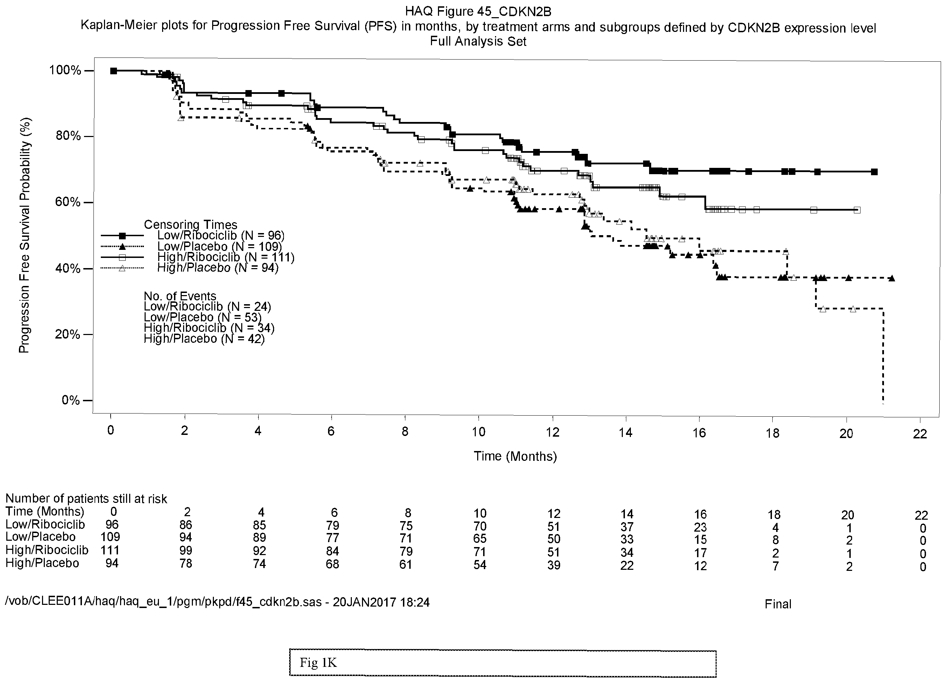

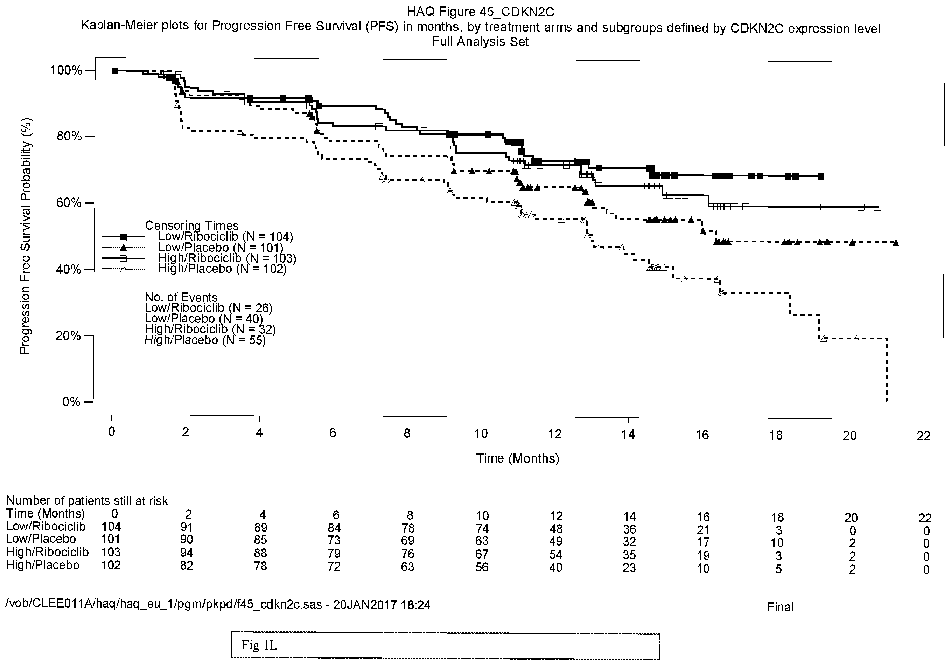

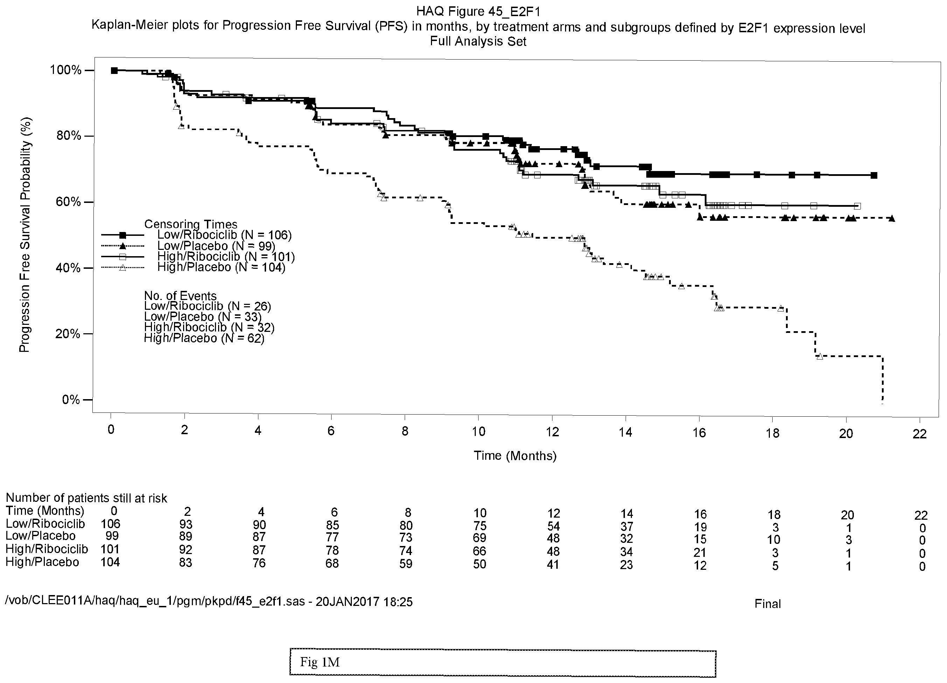

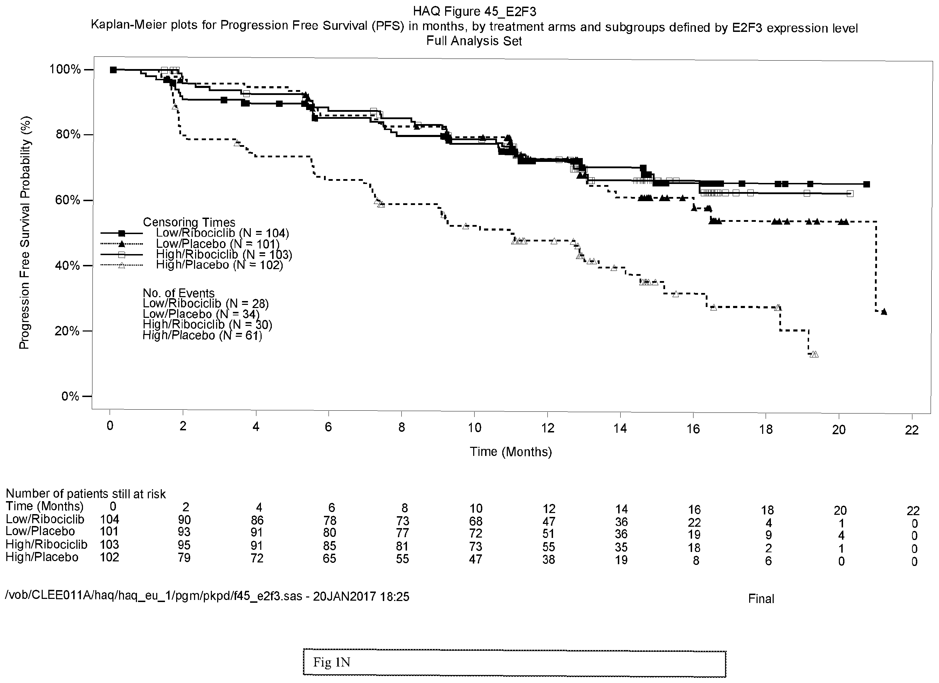

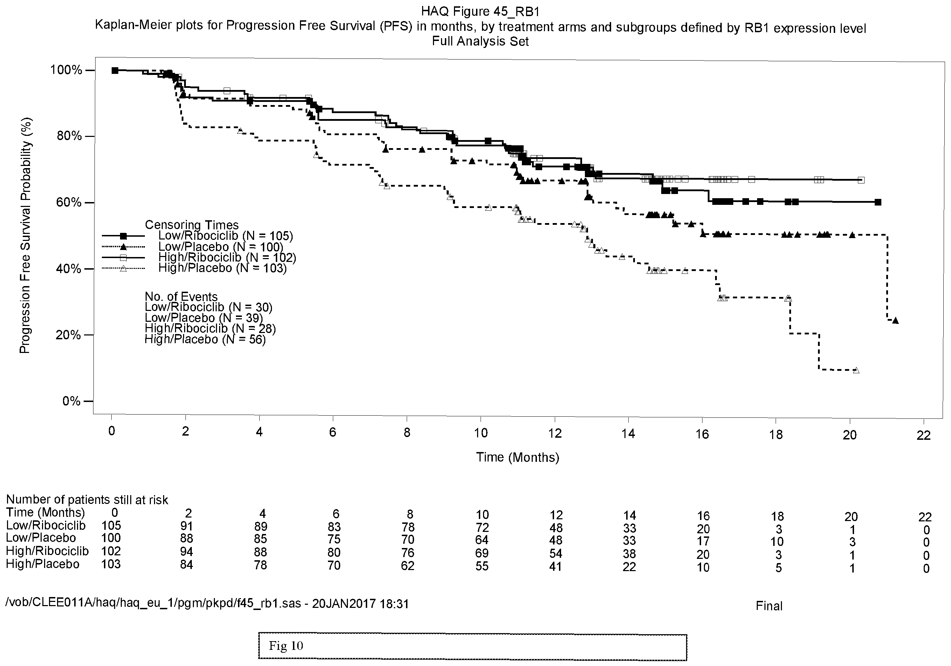

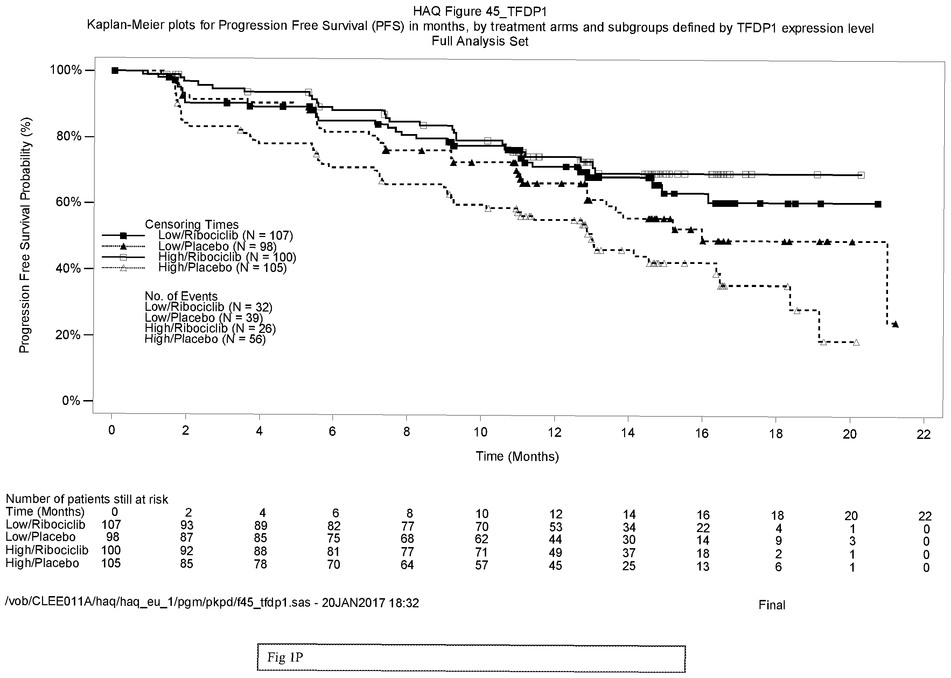

**Figure 1A-1Q** shows Kaplan Meier Curves for progression free survival (PFS) for various genes by treatment arm and subgroup defined by gene expression levels.

### DETAILED DESCRIPTION OF THE DISCLOSURE

There is an increasing body of evidence that suggests a patient's genetic profile can be determinative to a patient's responsiveness to a therapeutic treatment. Given the numerous therapies available to treat cancer, a determination of the genetic factors that influence, for example, prognosis, response to a particular drug, could be used to provide a patient with a personalized treatment regime. Such personalized treatment regimes offer the potential to maximize therapeutic benefit to the patient while minimizing related side effects that can be associated with alternative treatment regimes. Thus, there is a need to identify factors which can be used to predict prognosis of a patient and whether a patient is likely to respond to a particular therapy.

The biomarker(s) of the invention include one or more genes listed in Table 1. The level of expression of one or more gene(s) shown in Table 1 can include detecting nucleic acid products (e.g., RNA, mRNA, etc.) and/or polypeptide products (e.g., expressed proteins) obtained from a biological sample.

In another aspect, the invention includes the E2F3 gene listed in Table 2. The level of expression of one or more gene(s) shown in Table 2 can include detecting nucleic acid products (e.g., RNA, mRNA, etc.) and/or polypeptide products (e.g., expressed proteins) obtained from a biological sample.

By analyzing the expression level of one or more biomarkers identified in Table 1 or the biomarkers in Table 2 it is possible to predict the prognosis of cancer patients, e,g, breast cancer patients, and thus select patients most likely to respond to treatment with a particular drug(s). One embodiment not part of the invention can be used to identify breast cancer patients, who have a poor prognosis and thus would benefit from the treatment of a combination of an aromatase inhibitor, e.g., letrozole and a CDK 4/6 inhibitor, ribociclib.

### CDK 4/6 inhibitors

The cyclin-dependent kinase-4/6 (CDK 4/6) inhibitor can be any known CDK 4/6 inhibitor or a pharmaceutically acceptable salt, solvate, hydrate, cocrystal, or prodrug thereof. A number of CDK 4/6 inhibitors have been identified, including specific pyrido[2,3-d]pyrimidines, 2-anilinopyrimidines, diaryl ureas, benzoyl-2,4-diaminothiazoles, indolo[6,7-a]pyrrolo[3,4-c]carbazoles, and oxindoles (see P. S. Sharma, R. Sharma, R. Tyagi, Curr. Cancer Drug Targets 8 (2008) 53-75).

In one embodiment not part of the invention, the CDK 4/6 inhibitor is Palbociclib (tradename Ibrance^{®} by Pfizer, 1^{st} approved)

### (1) Palbociclib

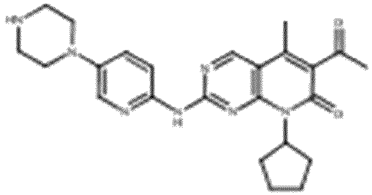

The commercial form is a free base, in capsule form for oral administration. The compound is disclosed in US Patent 6,936,612. The recommended dose of IBRANCE is a 125 mg capsule taken orally once daily for 21 consecutive days followed by 7 days off treatment to comprise a complete cycle of 28 days. IBRANCE is indicated for the treatment of HR-positive, HER2-negative advanced or metastatic breast cancer in combination with:
--an aromatase inhibitor as initial endocrine based therapy in postmenopausal women; or
--fulvestrant in women with disease progression following endocrine therapy.

In another embodiment, the CDK 4/6 is Ribociclib (tradename Kisqali^{®} by Novartis, 2nd approved).

### (2) Ribociclib (KISQALI)

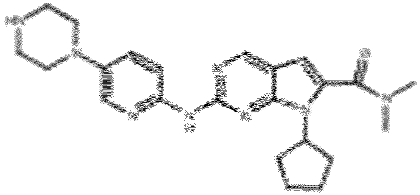

KISQALI^{®} is indicated in combination with an aromatase inhibitor as initial endocrine-based therapy for the treatment of postmenopausal women with hormone receptor (HR)-positive, human epidermal growth factor receptor 2 (HER2)negative advanced or metastatic breast cancer. The recommended dose of KISQALI is 600 mg (three 200 mg film-coated tablets) taken orally, once daily for 21 consecutive days followed by 7 days off treatment resulting in a complete cycle of 28 days. Coadminister KISQALI with letrozole 2.5 mg taken once daily throughout the 28-day cycle.

KISQALI is commercially available as tablet form for oral administration. The commercial salt form is ribociclib succinate.

Ribociclib is disclosed as Example 74 of US Patent 8,415,355. Ribociclib succinate salt form is described in US Patent 9,193,732.

In yet another embodiment not part of the invention, the CDK 4/6 inhibitor is Abemacicilib (by Lilly, to be approved soon as the 3^{rd} market entry).

### (3) Abemaciclib (LY2835219 by Eli Lilly)

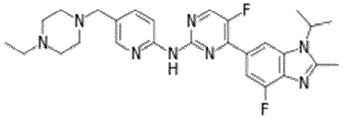

### Aromatase Inhibitors:

In one embodiment the aromatase inhibitor is letrozole (which is a non-steroidal inhibitor). In another embodiment, the aromatase inhibitor can be anastrazole (also non-steroidal inhibitor) or exemestane (irreversible steroidal inhibitor)

### Cancer:

The present disclosure can be used for prognostic purposes and/or can be used to select patients having cancer who would benefit from particular treatments. Any cancer can be screened according to the methods herein disclosed and include, but are not limited to, colon cancer, lung cancer, pancreatic cancer, gastric cancer, prostate cancer, and hepatocellular carcinoma, basal cell carcinoma, breast cancer, bone sarcoma, soft tissue sarcoma, chronic myeloid leukemia, acute myeloid leukemia, hematological cancer, medulloblastoma, rhabdomyosaracoma, neuroblastoma, pancreatic cancer, breast carcinoma, meningioma, glioblastoma, astrocytoma, melanoma, stomach cancer, esophageal cancer, biliary tract cancer, prostate cancer, small cell lung cancer, non-small cell lung cancer, glial cell cancer, multiple myeloma, colon cancer, neuroectodermal tumor, neuroendocrine tumor, mastocytoma and Gorlin syndrome. In a particular embodiment, the cancer is breast cancer. In one example, the cancer is metastatic breast cancer. In another example, the breast cancer is primary breast. In another example, the breast cancer is from a patient having HR-Positive, HER2-Negative or a patient having HR-Positive, HER2-Negative, post-menopausal, or a patient having HR-Positive, HER2-NegativeIn one embodiment, the patient having breast cancer is HR-Positive, HER2-Negative. In another embodiment, the patient having breast cancer is HR-Positive, HER2-Negative, post-menopausal. In yet another embodiment, the patient having breast cancer is HR-Positive, HER2-Negative, pre-menopausal.

### Preparation of Samples

Any appropriate test sample of cells taken from an individual having a proliferative disease can be used. Generally, the test sample of cells or tissue sample will be obtained from the subject with cancer by biopsy or surgical resection. A sample of cells, tissue, or fluid may be removed by needle aspiration biopsy. For this, a fine needle attached to a syringe is inserted through the skin and into the tissue of interest. The needle is typically guided to the region of interest using ultrasound or computed tomography (CT) imaging. Once the needle is inserted into the tissue, a vacuum is created with the syringe such that cells or fluid may be sucked through the needle and collected in the syringe. A sample of cells or tissue may also be removed by incisional or core biopsy. For this, a cone, a cylinder, or a tiny bit of tissue is removed from the region of interest. CT imaging, ultrasound, or an endoscope is generally used to guide this type of biopsy. More particularly, the entire cancerous lesion may be removed by excisional biopsy or surgical resection. In the present invention, the test sample is typically a sample of cells removed as part of surgical resection.

The test sample of, for example tissue, may also be stored in, e.g., RNAlater (Ambion; Austin Tex.) or flash frozen and stored at -80°C. for later use. The biopsied tissue sample may also be fixed with a fixative, such as formaldehyde, paraformaldehyde, or acetic acid/ethanol. The fixed tissue sample may be embedded in wax (paraffin) or a plastic resin. The embedded tissue sample (or frozen tissue sample) may be cut into thin sections. RNA or protein may also be extracted from a fixed or wax-embedded tissue sample or a frozen tissue sample. Once a sample of cells or sample of tissue is removed from the subject with cancer, it may be processed for the isolation of RNA or protein using techniques well known in the art and as described below.

An example of extraction of RNA from a biopsy taken from a patient with cancers can include, for example, guanidium thiocyanate lysis followed by CsCl centrifugation (Chirgwin, et al., Biochemistry 18:5294-5299, 1979). RNA from single cells may be obtained as described in methods for preparing cDNA libraries from single cells (see, e.g., Dulac, Curr. Top. Dev. Biol. 36:245, 1998; Jena, et al., J. Immunol. Methods 190:199, 1996). In one embodiment, the RNA population may be enriched for sequences of interest, as detailed in Tables 1 and 2. Enrichment may be accomplished, for example, by random hexamers and primer-specific cDNA synthesis, or multiple rounds of linear amplification based on cDNA synthesis and template-directed in vitro transcription (see, e.g., Wang, et al., Proc. Natl. Acad. Sci. USA 86:9717, 1989; Dulac, et al., supra; Jena, et al., supra).

The expression profile can be performed on a biopsy taken from a subject such as fresh tissue, frozen tissue, tissue processed in formalin (FFPE) or other fixatives.

The subject with a tumor or cancer will generally be a mammalian subject such as a primate. In an exemplary embodiment, the subject is a human.

### Detection of expression of the biomarker

In one example, the method includes determining expression level of one or more of the genes CCNA2, CCNE1, CDK2, CDK4, CDKN1A, CDKN2C, E2F1, E2F3, and RB1 or gene products. The gene sequences of interest can be detected using agents that can be used to specifically detect the expression level of the gene, for example, RNA transcribed from the gene or polypeptides encoded by the gene.

Analysis of the sequence of mRNA transcribed from a given gene can be performed using any known method in the art including, but not limited, to Northern blot analysis, nuclease protection assays (NPA), *in situ* hybridization, reverse transcription-polymerase chain reaction (RT-PCR), RT-PCR ELISA, TaqMan-based quantitative RT-PCR (probe-based quantitative RT-PCR), using Nanostring and SYBR green-based quantitative RT-PCR. In one example, detection of mRNA levels involves contacting the isolated mRNA with an oligonucleotide that can hybridize to mRNA encoded by an AI response marker. The nucleic acid probe can typically be, for example, a full-length cDNA, or a portion thereof, such as an oligonucleotide of at least 7, 15, 30, 50, or 100 nucleotides in length and sufficient to specifically hybridize under stringent conditions to the mRNA. Hybridization of an mRNA with the probe indicates that the marker in question is being expressed. In one format, the RNA is immobilized on a solid surface and contacted with a probe, for example by running the isolated RNA on an agarose gel and transferring the mRNA from the gel to a membrane, such as nitrocellulose. Amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers. PCR products can be detected by any suitable method including, but not limited to, gel electrophoresis and staining with a DNA-specific stain or hybridization to a labeled probe.

In one embodiment, the method includes: providing a nucleic acid probe comprising a nucleotide sequence, for example, at least 10, 15, 25 or 40 nucleotides, and up to all or nearly all of the coding sequence which is complementary to a portion of the coding sequence of a nucleic acid sequence of one or more of CCNA2, CCNE1, CDK2, CDK4, CDKN1A, CDKN2C, E2F1, E2F3, and RB1; obtaining a tissue sample from a mammal having a cancerous cell; contacting the nucleic acid probe under stringent conditions with RNA obtained from a biopsy taken from a patient with cancer (e.g., in a Northern blot, in situ hybridization assay, PCR etc); and determining the amount of hybridization of the probe with RNA. Nucleic acids may be labeled during or after enrichment and/or amplification of RNAs.

The biomarkers CCNA2, CCNE1, CDK2, CDK4, CDKN1A, CDKN2C, E2F1, E2F3, and RB1 are intended to also include naturally occurring sequences including allelic variants and other family members. The biomarkers of the invention also include sequences that are complementary to those listed sequences resulting from the degeneracy of the code and also sequences that are sufficiently homologous and sequences which hybridize under stringent conditions to the genes of the invention.

Conditions for hybridization are known to those skilled in the art and can be found in Current Protocols in Molecular Biology, John Wiley and Sons, N.Y. (1989), 6.3.1-6.3.6. A preferred, non-limiting example of highly stringent hybridization conditions are hybridization in 6 X sodium chloride/sodium citrate (SSC) at about 45 degrees centigrade followed by one or more washes in 0.2 X SSC, 0.1 percent SDS at 50-65 degrees centigrade. By "sufficiently homologous" it is meant a amino acid or nucleotide sequence of a biomarker which contains a sufficient or minimum number of identical or equivalent (e.g., an amino acid residue which has a similar side chain) amino acid residues or nucleotides to a second amino acid or nucleotide sequence such that the first and second amino acid or nucleotide sequences share common structural domains or motifs and/or a common functional activity. For example, amino acid or nucleotide sequences which share common structural domains have at least about 50 percent homology, at least about 60 percent homology, at least about 70 percent, at least about 80 percent, and at least about 90-95 percent homology across the amino acid sequences of the domains are defined herein as sufficiently homologous. Furthermore, amino acid or nucleotide sequences at least about 50 percent homology, at least about 60-70 percent homology, at least about 70-80 percent, at least about 80-90 percent, and at least about 90-95 percent and share a common functional activity are defined herein as sufficiently homologous.

The comparison of sequences and determination of percent homology between two sequences can be accomplished using a mathematical algorithim. A preferred, non-limiting example of a mathematical algorithim utilized for the comparison of sequences is the algorithm of Karlin and Altschul (1990) Proc. Natl. Acad. Sci. USA 87:2264-68, modified as in Karlin and Altschul (1993) Proc. Natl. Acad. Sci. USA 90:5873-77. Such an algorithm is incorporated into the NBLAST and XBLAST programs (version 2.0) of Altschul, et al. (1990) J. MoI. Biol. 215:403-10. BLAST nucleotide searches can be performed with the NBLAST program, score=100, wordlength=12 to obtain nucleotide sequences homologous to TRL nucleic acid molecules of the invention. BLAST protein searches can be performed with the XBLAST program, score=50, wordlength=3 to obtain amino acid sequences homologous to the protein sequences encoded by the genes listed in Table 1, or Table 2. To obtain gapped alignments for comparison purposes, Gapped BLAST can be utilized as described in Altschul et al., (1997) Nucleic Acids Research 25(17):3389-3402. When utilizing BLAST and Gapped BLAST programs, the default parameters of the respective programs (e.g., XBLAST and NBLAST) can be used. See http://www.ncbi.nlm.nih.gov. Another preferred, non-limiting example of a mathematical algorithim utilized for the comparison of sequences is the ALIGN algorithm of Myers and Miller, CABIOS (1989). When utilizing the ALIGN program for comparing amino acid sequences, a PAMl 20 weight residue table, a gap length penalty of 12, and a gap penalty of 4 can be used.

The present invention includes measuring the expression of one or more biomarkers CCNA2, CCNE1, CDK2, CDK4, CDKN1A, CDKN2C, E2F1, E2F3, and RB1 in a tumor biopsy taken from a subject suffering from cancer. The expression levels can be analyzed and used to generate a score which can be used to differentiate those patients having a tumor who will benefit from the treatment with the combination of CDK 4/6 inhibitor and an aromatase inhibitor compared with those patients who would benefit from other treatments such as treatment with an aromatase inhibitor alone.

In one embodiment, the method of the invention includes measuring any one of CCNA2, CCNE1, CDK2, CDK4, CDKN1A, CDKN2C, E2F1, E2F3, and RB1 listed in Table 1. In another embodiment, the method of the invention includes measuring at least two, at least three, at least four, at least five, at least six, at least seven, or at least eight genes, or at least nine biomarkers.

In one example, the level of expression of one gene, e.g., CCNA2 , from Table 1 is measured. In another example, using the claimed methods the level of expression of CCNE1 is measured. In yet another example, using the claimed methods the level of expression CDK2 is measured, etc

In another example, the level of expression of two genes, e.g., CCNA2 and CCNE1 from Table 1 is measured. In yet another example, the level of expression of three genes , CCNA2, CCNE1 and CDK2 from Table 1 is measured. In yet another example, the level of expression of four genes CCNA2, CCNE1, CDK2, and CDK4 from Table 1 is measured. In yet another example, the level of expression of five genes CCNA2, CCNE1, CDK2, CDK4, and CDKN1A from Table 1 is measured. In yet another example, the level of expression of five genes CCNA2, CCNE1, CDK2, CDK4, and CDKN1A, from Table 1 is measured. In yet another example, the level of expression of six genes CCNA2, CCNE1, CDK2, CDK4, CDKN1A, and CDKN2C from Table 1 is measured. In still yet another example, the level of expression of seven genes CCNA2, CCNE1, CDK2, CDK4, CDKN1A, CDKN2C and E2F1 is measured. In yet another example, the level of expression of eight genes CCNA2, CCNE1, CDK2, CDK4, CDKN1A, CDKN2C, E2F1, and E2F3 is measured. In yet another embodiment, the expression of at least nine genes CCNA2, CCNE1, CDK2, CDK4, CDKN1A, CDKN2C, E2F1, E2F3, and RB1 is measured. Any combination of said genes of Table 1 can be measured and an increase in expression relative to a control is indicative that that patient should be selected for therapy for the combination of the CDK 4/6 inhibitor ribociclib and an aromatase inhibitor such as letrozole.

**Table 1**

| **Gene Name** | **Accession # Uni Gene ID** |
|---|---|
| **CCNA2,** | **UGID: NM_001237** |
| **CCNE1,** | **UGID: NM_001238** |
| **CDK2,** | **UGID: NM_001798** |
| **CDK4,** | **UGID: NM_000075** |
| **CDKN1A,** | **UGID: NM_000389** |
| **CDKN2B,** | **UGID: NM_004936** |
| **CDKN2C,** | **UGID: NM_001262** |
| **E2F1,** | **UGID: NM_005225** |
| **RB1** | **NM_000321** |
| **E2F3** | **NM_001949** |

The biomarkers of the invention also include any combination of genes identified in Table 1 whose level of expression or gene product serves as a predictive marker or biomarker.

In the method of the invention the level of expression of one or more genes as described above is measured and analyzed and used to generate a score which can be used to select those subjects having a good prognosis or those having a bad prognosis and/or select patients who are more likely to benefit from treatment with the combination of a CDK 4/6 inhibitor and an aromatase inhibitor.

Alternatively, the biomarkers listed in Table 2 can be used to select patients who would most likely benefit from treatment with a CDK 4/6 inhibitor and an aromatase inhibitor.

**Table 2**

| **Gene Name** | **Accession # Uni Gene ID** |
|---|---|
| **E2F3** | **NM_001949** |
| **CDKN2A** | **NM_000077** |

In the methods of the invention, the expression of each biomarker is measured and typically will be converted into an expression value after normalization by the expression level of the single control gene. These expression values then will be used to generate a score which is then compared against a cut-off to select those subjects having a good prognosis or those having a bad prognosis and/or select patients who are more likely to benefit from treatment with the combination of a CDK 4/6 inhibitor and an aromatase inhibitor.

The biomarkers of the invention can be measured using any method known in the art such as reverse Transcriptase PCR (RT-PCR). The method includes isolating mRNA using any technique known in the art, e.g., by using a purification kit, buffer set and protease from commercial manufacturers, such as Qiagen. The reverse transcription step is typically primed using specific primers, random hexamers, or oligo-dT primers, depending on the circumstances and the goal of expression profiling and the cDNA derived can then be used as a template in the subsequent PCR reaction. TaqMan(R) RT-PCR can then be performed using , e.g., commercially available equipment.

A more recent variation of the RT-PCR technique is the real time quantitative PCR, which measures PCR product accumulation through a dual-labeled fluorigenic probe (e.g., using TaqMan(R) probe). Real time PCR is compatible both with quantitative competitive PCR, where internal competitor for each target sequence is used for normalization, and with quantitative comparative PCR using a normalization gene contained within the sample, or a housekeeping gene for RT-PCR. For further details see, e.g. Held et al, Genome Research 6:986-994 (1996).

In another example, microarrays are used which include one or more probes corresponding to one or more of genes CCNA2, CCNE1, CDK2, CDK4, CDKN1A, CDKN2B, CDKN2C, E2F1, E2F3, and RB1. The method described above results in the production of hybridization patterns of labeled target nucleic acids on the array surface. The resultant hybridization patterns of labeled nucleic acids may be visualized or detected in a variety of ways, with the particular manner of detection selected based on the particular label of the target nucleic acid. Representative detection means include scintillation counting, autoradiography, fluorescence measurement, calorimetric measurement, light emission measurement, light scattering, and the like.

In another example, a TaqMan^{®} Low Density Array ( TLDA) card can be used which can include one or more probes corresponding to one or more of genes CCNA2, CCNE1, CDK2, CDK4, CDKN1A, CDKN2B, CDKN2C, E2F1, E2F3, and RB1. This method uses a microfluidic card that performs simultaneous real time PCR reactions.

In one example, the method of detection utilizes an array scanner that is commercially available (Affymetrix, Santa Clara, Calif.), for example, the 417.TM. Arrayer, the 418.TM. Array Scanner, or the Agilent GeneArray.TM. Scanner. This scanner is controlled from a system computer with an interface and easy-to-use software tools. The output may be directly imported into or directly read by a variety of software applications. Scanning devices are described in, for example, U.S. Pat. Nos. 5,143,854 and 5,424,186.

As used herein, the control for comparison can be determined by one skilled in the art. In one aspect, the control is determined by choosing a value that serves as a cut-off value. For example, the value can be a value that differentiates between e.g., those test samples that have an increase in expression of a biomarker of the invention from those that do not show an increase in expression. In another example, the gene expression profile of a biomarker of the invention is compared to a control (presence of expression of the biomarker in a sample taken from a healthy person).

### Detecting expression of the biomarker gene product

Detecting for the presence of a protein product encoded by one or more of CCNA2, CCNE1, CDK2, CDK4, CDKN1A, CDKN2C, E2F1, E2F3, and RB1 can be done by using any appropriate method known in the art. For example, an agent of interest that can be used to detect a particular protein of interest, for example using an antibody. The method for producing polyclonal and/or monoclonal antibodies that specifically bind to polypeptides useful in the present invention is known to those of skill in the art and may be found in, for example, Dymecki, et al., (J. Biol. Chem. 267:4815, 1992); Boersma and Van Leeuwen, (J. Neurosci. Methods 51:317, 1994); Green, et al., (Cell 28:477, 1982); and Arnheiter, et al., (Nature 294:278, 1981). In one embodiment, an immunoassay can be used to quantitate the levels of proteins in cell samples. The invention is not limited to a particular assay procedure, and therefore, is intended to include both homogeneous and heterogeneous procedures.

Exemplary immunoassays that may be conducted according to the invention include fluorescence polarization immunoassay (FPIA)₅ fluorescence immunoassay (FIA), enzyme immunoassay (EIA), nephelometric inhibition immunoassay (NIA), enzyme-linked immunosorbent assay (ELISA), and radioimmunoassay (RIA). An indicator moiety, or label group, may be attached to the subject antibodies and is selected so as to meet the needs of various uses of the method that are often dictated by the availability of assay equipment and compatible immunoassay procedures. General techniques to be used in performing the various immunoassays noted above are known to those of ordinary skill in the art.

Alternatively other methods can be used such as Western blot analysis that includes electrophoretically separating proteins on a polyacrylamide gel, and after staining the separated proteins, the relative amount of each protein can be quantified by assessing its optical density.

Alternatively, other methods such as dot-blot assays, FACS or immunohistochemistry can be used.

Typically, antibodies generated against the biomarkers of the invention can be used for visualizing for the presence of a protein of interest can be labeled, for example, using a reporter molecule such as fluorophores, enzymes, biotin, chemiluminescent molecules, bioluminescent molecules, digoxigenin, avidin, streptavidin or radioisotopes.

In yet another embodiment, the invention contemplates using a panel of antibodies that are generated against the marker polypeptides of this invention.

### Data analysis

To facilitate the sample analysis operation, the data obtained by the reader from the device may be analyzed using a digital computer. Typically, the computer will be appropriately programmed for receipt and storage of the data from the device, as well as for analysis and reporting of the data gathered, for example, subtraction of the background, verifying that controls have performed properly, normalizing the signals, interpreting fluorescence data to determine the amount of hybridized target, normalization of background, and the like.

Specifically the result can be cast in a transmittable form of information that can be communicated or transmitted to other researchers or physicians or genetic counselors or patients. Such a form can vary and can be tangible or intangible. The result in the individual tested can be embodied in descriptive statements, diagrams, photographs, charts, images or any other visual forms. For example, images of gel electrophoresis of PCR products can be used in explaining the results. Statements regarding levels of gene expression and levels of protein are also useful in indicating the testing results. These statements and visual forms can be recorded on a tangible media such as papers, computer readable media such as floppy disks, compact disks, etc., or on an intangible media, e.g., an electronic media in the form of email or website on internet or intranet. In addition, the result can also be recorded in a sound form and transmitted through any suitable media, e.g., analog or digital cable lines, fiber optic cables, etc., via telephone, facsimile, wireless mobile phone, internet phone and the like. All such forms (tangible and intangible) would constitute a "transmittable form of information". Thus, the information and data on a test result can be produced anywhere in the world and transmitted to a different location. For example, when detecting the level of mRNA in an assay conducted offshore, the information and data on a test result may be generated and cast in a transmittable form as described above. The test result in a transmittable form thus can be imported into the U.S. Accordingly, the present disclosure also encompasses a method for producing a transmittable form of information containing levels of biomarker expression and/or levels of biomarker protein/activit in a cancer patient. This form of information is useful for predicting the responsiveness of a cancer patient, for selecting a course of treatment based upon that information, and for selectively treating a patient based upon that information.

### Kits

Not part of the invention, the present specification provides kits for determining the expression level of the biomarkers described herein. The kits may be useful for determining who will benefit from treatment with a CDK 4/6 inhibitor and an aromatase inhibitor. A kit can comprise probes of genes identified in Table 1 can be used to measure gene expression of a test sample. In one embodiment, the kit comprises a computer readable medium which includes expression profile analysis software capable of being loaded into the memory of a computer system and which can convert the measured expression values into a risk score. A kit may further comprise nucleic acid controls, buffers, and instructions for use.

### Administration

CDK 4/6 can be administered in therapeutically effective amounts via any of the usual and acceptable modes known in the art, either singly or in combination with one or more therapeutic agents such as an aromatase inhibitor, e.g., letrozole. A therapeutically effective amount may vary widely depending on the severity of the disease, the age and relative health of the subject, the potency of the compound used and other factors.

The methods of the invention can be used to select those cancer patients, breast cancer patients, who would benefit most from treatment with a the combination of a CDK 4/6 inhibitor and an aromatase inhibitor, e.g., letrozole. The therapeutic agents of CDK 4/6 inhibitor and an aromatase inhibitor can either be a fixed combination in one dosage unit form or a kit of parts for the combined administration where the CDK 4/6 inhibitor and the aromatase inhibitor may be administered independently at the same time or separately within time intervals.

One skilled in the art will recognize many methods and materials similar or equivalent to those described herein, which could be used in the practice of the present invention. Indeed, the present invention is in no way limited to the methods and materials described.

### EXAMPLES

### Example 1: MONALEESA-2, a randomized double-blind, placebo-controlled study of LEE011 in combination with letrozole for the treatment of postmenopausal women with hormone receptor positive, HER2-negative, advanced breast cancer, who received no prior therapy for advanced disease.

### Methods

Postmenopausal
HR+ HER2- mBC

No prior therapy for
mBC

De novo or > 12 months
post-(neo) adjuvant
therapy with letrozole
or anastrozole

R
A
N
D
O
M
I
Z
A
T
I
O
N

LEE011
600mg (day 1 to 21
in a 28 day cycle) +
letrozole (daily)
(N = 325)

OR

Placebo (day 1 to 21
in a 28 day cycle) +
letrozole (daily)
(N = 325)

Discontinuation
due to
toxicity

Continue
efficacy
assessments

Survival

Discontinuation
due to
progression

Survival

Figure: Study design – Study CLEE011A2301

### Study Participants

The patient population consisted of postmenopausal women with HR-positive, HER2- negative, advanced breast cancer, who had previously received no prior therapy for their advanced breast cancer.

### The main inclusion criteria were the following:

• Adult postmenopausal women (≥ 18 years) at the time of informed consent.
• Women with advanced (loco regionally recurrent or metastatic) breast cancer not amenable to curative therapy.
• Postmenopausal status was defined either by:
   - Prior bilateral oophorectomy
   - Age ≥ 60
   - Age < 60 and amenorrhea for 12 or more months (in the absence of chemotherapy, tamoxifen, toremifen, or ovarian suppression) and follicle stimulating hormone (FSH) and estradiol in the postmenopausal range per local normal range.
• Histological and/or cytological confirmation of estrogen receptor (ER)-positive - and/or progesterone receptor positive breast cancer by local laboratory.
• Patients diagnosed with HER2-negative breast cancer defined as a negative in situ hybridization test or an Immunohistochemistry (IHC) status of 0, 1+ or 2+. If IHC was 2+, a negative in situ hybridization (fluorescent in situ hybridization [FISH], chromosome in situ hybridization [CISH], or silver-enhanced in situ hybridization [SISH]) test was required by local laboratory testing.

### Patient had either:

- Measurable disease, i.e., at least one measurable lesion as per RECIST 1.1 criteria (Tumor lesions previously irradiated or subjected to other loco regional therapy were only to be considered measurable if disease progression at the treated site after completion of therapy was clearly documented)
   OR
- At least one predominantly lytic bone lesion (Patients with only one predominantly lytic bone lesion that was previously irradiated were eligible if there was documented evidence of disease progression of the bone lesion after irradiation).
- ECOG performance status 0 or 1

### The main exclusion criteria were:

• Patients who received any CDK4/6 inhibitor previously.
• Patients with inflammatory breast cancer.
• Patients who received any prior systemic anti-cancer therapy (including hormonal therapy and chemotherapy) for advanced breast cancer
• Patients who received (neo) adjuvant therapy for breast cancer were eligible. If the prior neo (adjuvant) therapy included letrozole or anastrozole the disease free interval had to be greater than 12 months from the completion of treatment until randomization. Patients who received ≤ 14 days of letrozole or anastrozole for advanced disease prior to randomization were allowed.
• Any prior (neo) adjuvant anti-cancer therapy had to be stopped at least 5 half-lives or 7 days, whichever was longer, before randomization
• Patients currently receiving other anti-cancer therapy.
• Patients with central nervous system (CNS) metastases.
• Patients who had active cardiac disease or a history of cardiac dysfunction including any of the following:
   - History of angina pectoris, symptomatic pericarditis, or myocardial infarction within 12 months prior to study entry
   - History of documented congestive heart failure (New York Heart Association functional classification III-IV)
   - Documented cardiomyopathy
   - Left Ventricular Ejection Fraction (LVEF) <50% as determined by Multiple Gated acquisition (MUGA) scan or echocardiogram (ECHO)
   - History of any cardiac arrhythmias, e.g., ventricular, supraventricular, nodal arrhythmias, or conduction abnormality in the previous 12 months.
   - On Screening, any of the following cardiac parameters: bradycardia (heart rate <50 at rest), tachycardia (heart rate >90 at rest), pulse rate interval >220 msec, QRS interval >109 msec, or QTcF >450 msec.
   - Systolic blood pressure >160 or <90 mmHg.
• Patients that were currently receiving any of the following medications and could not be discontinued seven days prior to the start of the treatment:
   ∘ Known strong inducers or inhibitors of CYP3A4/5.
   ∘ Those with a known risk to prolong the QT interval or induce Torsades de Pointes.
   ∘ Those with a narrow therapeutic window and are predominantly metabolized through CYP3A4/5.
   ∘ Herbal preparations/medications.
   ∘ Dietary supplements (except vitamins).
• Patients that were currently receiving or those who had received systemic corticosteroids ≤ 2 weeks prior to starting study drug, or who have not fully recovered from side effects of such treatment.
Note: The use of following corticosteroids was permitted: single doses of topical applications (e.g., for rash), inhaled sprays (e.g., for obstructive airways diseases), and eye drops or local injections (e.g., intra-articular).

### Treatments

Arm A: Ribociclib (600 mg once daily, days 1-21 of a 28-day cycle) + letrozole (2.5 mg once daily, days 1-28 of a 28-day cycle).

Arm B: Placebo (once daily, days 1-21 of a 28-day cycle) + letrozole (2.5 mg once daily days 1-28 of a 28-day cycle continuously).

The ribociclib/placebo dose could be reduced to 400 mg (first reduction) and 200 mg (second reduction), whereas no dose modification for letrozole was planned in the study.

Patients received study treatment until disease progression, unacceptable toxicity, death, or discontinuation from the study treatment for any other reason.

### Objectives

### Primary objective

The primary objective was to compare progression-free survival (PFS) in patients treated with ribociclib and letrozole to that of patients treated with placebo and letrozole, according to investigators' review.

### Secondary objectives

The key secondary objective of the study was to compare the two treatment arms with respect to overall survival (OS).

Other secondary objectives were:
- To evaluate the two treatment arms with respect to overall response rate (ORR) and clinical benefit rate (CBR).
- To evaluate the two treatment arms with respect to time to deterioration of Eastern Cooperative Oncology Group (ECOG) performance status.
- To evaluate the safety and tolerability of ribociclib in combination with letrozole.
- To evaluate patient reported outcomes (PROs) for health-related quality of life (QoL) in the two treatment arms.

### Exploratory objectives

Time to response, duration of response, exposure for ribociclib and letrozole, effects on estradiol suppression for combination vs. monotherapy, exposure-response relationship, hospital resource utilization, signaling pathway alterations, circulating DNA, mechanisms of resistance.

### Outcomes/endpoints

PFS was determined through investigators review of radiology data using RECIST version 1.1 criteria. Tumour assessments (CT with iv contrast or without iv contrast combined with MRI, visual assessment, photograpy) were made every 8 weeks during the first 18 months, and every 12 weeks thereafter until disease progression and at end-of-treatment.

PFS was defined as the time from the date of randomization to the date of first documented disease progression or death due to any cause. Discontinuation for non-RECIST progression was not counted as a PFS event. If a patient started other anti-neoplastic therapy or had two or more missing tumour assessments, PFS was censored at the last adequate tumour assessment, regardless of subsequent events.

A blinded independent review committee (BIRC) was utilized for review of radiology (and photography) data. At the start of the study, the BIRC consisted of a single radiology reader and an oncologist who would review all data for select patients where radiological progression could not be fully confirmed. The decision regarding patient management remained with the investigator.

Overall survival (OS) was a key secondary endpoint. Overall survival (OS) was defined as the time from date of randomization to date of death due to any cause.

Other secondary efficacy variables were ORR, CBR, and ECOG performance status. ORR was defined as the proportion of patients with best overall response of confirmed CR or PR according to RECIST 1.1, and CBR defined as the proportion of patients with a best overall response of confirmed CR or PR or stable disease (SD) lasting 24 weeks or longer, according to RECIST 1.1 criteria.

Patient-reported outcomes (PROs) were evaluated using the European Organization for Research and Treatment of Cancer (EORTC) QLQ-C30 (version 3.0) questionnaire along with the disease-specific breast cancer module (EORTC QLQ-BR23, version 1.0) and the EuroQoL 5-level instrument (EQ-5D-5L, Version 4.0) to explore impacts on health-related quality of life, functioning, disease symptoms, and treatment-related side effects.

### Sample size

It was initially planned to randomise 500 patients. Within a study protocol amendment (amendment 2, 25-Nov-2014) the sample size of the study was increased to 650 in order to better characterize the effect of ribociclib and letrozole on overall survival.

It was estimated that the median duration of PFS in the control group (placebo plus letrozole) would be 9.0 months, and treatment with test treatment arm (ribociclib plus letrozole) would result in a 33% reduction in the hazard rate (corresponding to an increase in median PFS to 13.43 months).

If the true hazard ratio was 0.67 (under alternative hypothesis), a total of 302 PFS events were required to have 93.5% power at a one-sided overall 2.5% level of significance to reject the null hypothesis (HR=1) using a log-rank test and a 2-look group sequential design with Haybittle-Peto to determine efficacy boundary for the interim analysis. Considering a recruitment period of approximately 16 months at a uniform rate of 37 patients/month 592 patients needed to be randomized to the two treatment arms in a 1:1 ratio. Assuming about 10% patients were to be lost to follow-up for PFS, a total of 650 patients needed to be randomized. Given the above assumptions, it was estimated that the 302th PFS event were to be observed at approximately 20 months from the date of first patient randomized in the study.

The median OS in the letrozole only arm was expected to be around 34 months. It was hypothesized that test treatment arm (ribociclib plus letrozole) resulted in a 28% reduction in the hazard rate for overall survival (corresponding to an increase in median survival to 47.2 months). If the true hazard ratio was 0.72 (under alternative hypothesis), a total of 400 deaths needed to be observed to have 90% power at a one-sided overall 2.5% level of significance to reject the null hypotheses (HR=1) using a log-rank test and a 4-look group sequential design.

### Randomisation

Eligible patients were randomized using IRT system in a 1:1 ratio.

Randomization was stratified by the presence of liver and/or lung metastases (yes versus no).

### Blinding (masking)

Double-blind study.

### Statistical methods

This was a superiority trial, powered to detect a difference (at least) corresponding to a median PFS of 13.43 months (experimental arm) vs. 9 months (control arm), with 93.5% probability (HR 0.67, one-sided overall 2.5% level of significance, log-rank test). This also involved a 2-look group sequential design with an interim analysis for superiority. The interim analysis was planned to be performed after approximately 211 events, requiring a p < 0.0000129 for conclusion of superiority, whereas the final analysis was planned after 302 events, requiring a p-value of 0.02499 for statistical significance.

Assuming a median OS of 47.2 months for the experimental arm vs. 34 for the control arm (HR of 0.72), 400 events would be necessary for 90% power to detect a difference (one-sided overall 2.5% level of significance, log-rank test, 4-look group sequential design). The four OS analyses were planned to be performed after 76 (interim PFS analysis), 120 (final PFS analysis), 300 and 400 deaths.

Investigators and patients will remain blinded to study treatment and all patients will continue to be followed for OS until the final OS analysis (or earlier if OS reaches statistical significance at any of the interim analyses).

A hierarchical testing strategy, where OS was to be statistically evaluated only if the primary efficacy endpoint PFS was statistically significant, was used to control the overall type-I error rate.

All efficacy analyses were performed using the Full Analysis Set (FAS) which consisted of all randomised patients. According to the intent to treat principle, patient data were analysed according to the treatment and stratum they had been assigned to at randomisation. Efficacy analyses included all data observed in patients from FAS regardless whether it was observed on-treatment or after the study treatment discontinuation till the analysis cut-off date.

As a supportive analysis, the primary PFS analysis was to be repeated using data from BIRC assessments using FAS and using the same conventions as for the primary analysis.

Several sensitivity analyses were planned to assess the overall robustness of the primary efficacy results. These analyses included repeating the primary efficacy analysis using the Per Protocol Set, using an unstratified log-rank test and using different censoring rules.

### Results

### Participant flow

Enrolment
Assessed for eligibility
(n= 958)
Excluded (n= 290)
- Screen failures (n= 252)
- Subject decision (n= 32)
- Physician decision (n= 4)
- Adverse event (n= 1)
- Lost to follow-up (n= 1)
Randomised (n = 668)
Allocation
Allocated to ribociclib + letrozole (n = 334)
Received ribociclib + letrozole (n= 334)
Allocated to placebo + letrozole (n =334)
Received placebo + letrozole (n= 330)
Did not receive placebo + letrozole (n= 4)
- Physician decision (n= 3)
- Subject decision (n= 1)
Follow-up
Treatment ongoing (n= 195)
End of treatment (n= 139)
- Progressive disease (n= 87)
- Adverse event (n = 25)
- Subject decision (n= 12)
- Physician decision (n= 10)
- Protocol deviation (n= 3)
- Death (n = 2)
Post treatment follow-up (n= 13)
- Continued follow-up (n= 10)
- Progressive disease (n= 3)
Survival follow-up (n= 113)
Treatment ongoing (n= 154)
End of treatment (n= 176)
- Progressive disease (n= 146)
- Adverse event (n = 7)
- Subject decision (n= 13)
- Physician decision (n= 13)
- Protocol deviation (n= 1)
- Death (n = 0)
Post treatment follow-up (n= 6)
- Continued follow-up (n= 3)
- Progressive disease (n= 2)
- Death (n=1)
Survival follow-up (n= 161)
Analysis
PFS, investigators review (n= 334)
PFS events (n= 93)
- Progression (n= 89)
- Death (n= 4)
Censored (n= 241)
- Followed without event (n= 194)
- Inadequate assessments (n= 21)
- New cancer therapy (n= 13)
- Withdrew consent (n= 10)
- Event after ≥2 missing tumour assessments
(n= 3)
Overall survival (n= 334)
Deaths (n= 23)
Censored (n=311)
- Alive (n= 296)
- Lost to follow-up (n= 15)
PFS, investigators review (n= 334)
PFS events (n= 150)
- Progression (n= 150)
- Death (n= 0)
Censored (n= 184)
- Followed without event (n= 143)
- Inadequate assessments (n= 12)
- New cancer therapy (n= 18)
- Withdrew consent (n= 10)
- Event after ≥2 missing tumour assessments (n= 1)
Overall survival (n= 334)
Deaths (n= 20)
Censored (n=314)
- Alive (n= 297)
- Lost to follow-up (n= 17)

### Example 2: Sample Collection & Accessioning

Biomarker analysis was performed on formalin-fixed, paraffin-embedded (FFPE) tissue tumor tissue samples provided by clinical study sites.

Analytical Methods Summary: Gene Expression by NanoString Technology Using Counter Gene Expression Assays offered by NanoString^{®} Technologies, Inc. which are designed to provide an ultra-sensitive, reproducible and highly multiplexed method for gene expression profiling across all levels of biological expression. The methodology allows for direct detection of mRNAs with molecular barcodes called nCounter Reporter Probes, without the use of reverse transcription or amplification. The nCounter GX Human Cancer Reference Kit (NanoString, catalog number150091) is a comprehensive set of 230 human cancer-related genes and six internal reference genes. These genes represent a broad range of relevant cancer-related processes.

### RNA Extraction

Four to eight 5 ± 1 µm FFPE sections were cut for each sample containing greater than or equal to 10% tumor content, as determined by pathologist review of hematoxylin and eosin (H&E) stained adjacent tissue section. Sections were dewaxed using the Tissue-Tek Prisma automated stainer and macro-dissected according to the tumor region specified during the pathologist review of H&E stained tissue section. RNA was extracted from tissue scrapes using the manual spin-based procedure for the Qiagen RNEasy FFPE kit following manufacturer's instructions.

Following extraction, RNA was quantified using a NanoDrop 8000 Spectrophotometer.

### NanoString Reaction Set Up

Hybridization reactions were prepared according to the NanoString protocol for gene expression using legacy reagents using a minimum starting RNA input of 50 ng in a maximum volume of 10 µL. Hybridization proceeded for 20 ± 4 hours at 65°C, after which samples were purified and analyzed using the NanoString nCounter^{®} GEN2 Prep Station and Digital Analyzer.

### NanoString Data Analysis

For each sample, RCC files (.rcc) containing raw count data were obtained from the NanoString nCounter^{®} GEN2 Digital Analyzer and loaded onto NanoString nSolver^{™} v1.1 for data processing.

ERCC positive control data normalization was performed on all samples passing quality control criteria using the Positive Control Normalization function of nSolver^{™} v1.1. The resulting ERCC positive control normalized values per vendor specification were transferred to clinical database for further analysis.

The analysis set includes 410 patients from the ITT (Intent to Treat) patients with NanoString assessments in the CLEE011A2301/MONALEESA-2 study. Subgroup analyses were performed to identify if baseline RNA gene expressions of the single gene were associated with Ribociclib treatment efficacy.

Patients were classified into high (> median) and low (<= median) biomarker subgroups by the median expression levels of each single gene. Kaplan-Meier estimates of median progression free survival (PFS) time for both treatment arms were estimated within each subgroup. Cox Proportional Hazards models stratified by liver and / or lung metastasis per IRT (Interactive Response Technologies) were used to calculate the hazard ratios as well as 95% confidence intervals for ribociclib 600mg + letrozole 2.5mg versus placebo + letrozole 2.5mg in each subgroup. Log-rank tests were used to test for the difference in the survival distributions among treatment and gene expression subgroups.

Correlation of efficacy (PFS) and gene expressions of the following genes that are directly involved in cell cycle progression were evaluated. These genes are: CDKN1A, CDKN2A, CDKN2B, CDKN2C, CCND1, CCND2, CCND3, CCNA2, E2F1, E2F3, TFDP1 and RB1. As shown below, these exploratory analyses with above-mentioned CDK4/6 pathway related biomarkers indicated that all patients benefited from the combination treatment of ribociclib and letrozole. The PFS of the letrozole alone arm varied with several of these biomarkers, however, the combination of ribociclib plus letrozole consistently improved PFS.

**Table 3: Subgroup analysis of association of PFS vs. mRNA expression levels of specified individual gene measured by NanoString technology. Low and high levels of mRNA expression was defined by median cut-off for each gene.**

| **Biomarker subgroup** | **Ribociclib + letrozole** | | | **Placebo + letrozole** | | | **Ribociclib + letrozole vs. placebo + letrozole** |
|---|---|---|---|---|---|---|---|
| | **n** | **No of events** | **Median PFS** | **n** | **No of events** | **Median PFS** | **HR (95% Cl)** |
| CCNA2 high (>170.7) | 101 | 30 | NE | 104 | 61 | 11.07 | 0.411 (0.264,0.640) |
| CCNA2 low (<=170.7) | 106 | 28 | NE | 99 | 34 | 20.99 | 0.773 (0.466,1.282) |
| CCND1 high (>2001.6) | 105 | 30 | NE | 100 | 48 | 14.13 | 0.597 (0.375,0.950) |
| CCND1 low (<=2001.6) | 102 | 28 | NE | 103 | 47 | 15.97 | 0.497 (0.309,0.798) |
| CCND2 high (>265.8) | 109 | 30 | NE | 96 | 48 | 13.37 | 0.461 (0.291,0.730) |
| CCND2 low (<=265.8) | 98 | 28 | NE | 107 | 47 | 15.97 | 0.637 (0.397,1.023) |
| CCND3 high (>258.9) | 107 | 33 | NE | 98 | 54 | 12.85 | 0.469 (0.303,0.726) |
| CCND3 low (<=258.9) | 100 | 25 | NE | 105 | 41 | 16.36 | 0.635 (0.385,1.048) |
| CCNE1 high (>45.7) | 103 | 33 | NE | 102 | 59 | 12.71 | 0.454 (0.295,0.697) |
| CCNE1 low (<=45.7) | 104 | 25 | NE | 101 | 36 | 20.99 | 0.689 (0.412,1.151) |
| CDK2 high (>136.4) | 102 | 34 | NE | 103 | 60 | 12.85 | 0.508 (0.332,0.779) |
| CDK2 low (<=136.4) | 105 | 24 | NE | 100 | 35 | NE | 0.603 (0.358,1.017) |
| CDK4 high (>433.2) | 106 | 32 | NE | 99 | 53 | 12.85 | 0.476 (0.305,0.745) |
| CDK4 low (<=433.2) | 101 | 26 | NE | 104 | 42 | 20.99 | 0.586 (0.357,0.963) |
| CDK6 high (>107.3) | 99 | 30 | NE | 106 | 52 | 13.04 | 0.527 (0.334,0.832) |
| CDK6 low (<=107.3) | 108 | 28 | NE | 97 | 43 | 16.36 | 0.546 (0.338,0.883) |
| CDKN1A high (>404.6) | 105 | 31 | NE | 100 | 55 | 13.01 | 0.458 (0.293,0.717) |
| CDKN1A low (<=404.6) | 102 | 27 | NE | 103 | 40 | 20.99 | 0.655 (0.400,1.072) |
| CDKN2A high (>31.5) | 106 | 33 | NE | 99 | 49 | 13.37 | 0.568 (0.364,0.886) |
| CDKN2A low (<=31.5) | 101 | 25 | NE | 104 | 46 | 15.97 | 0.522 (0.320,0.853) |
| CDKN2B high (>178.2) | 111 | 34 | NE | 94 | 42 | 15.97 | 0.674 (0.426,1.065) |
| CDKN2B low (<=178.2) | 96 | 24 | NE | 109 | 53 | 13.63 | 0.447 (0.276,0.725) |
| CDKN2C high (>182.0) | 103 | 32 | NE | 102 | 55 | 12.98 | 0.502 (0.323,0.780) |
| CDKN2C low (<=182.0) | 104 | 26 | NE | 101 | 40 | 16.36 | 0.608 (0.371,0.997) |
| E2F1 high (>106.7) | 101 | 32 | NE | 104 | 62 | 11.43 | 0.463 (0.301,0.713) |
| E2F1 low (<=106.7) | 106 | 26 | NE | 99 | 33 | NE | 0.725 (0.433,1.214) |
| E2F3 high (>95.3) | 103 | 30 | NE | 102 | 61 | 11.04 | 0.379 (0.244,0.589) |
| E2F3 low(<=95.3) | 104 | 28 | NE | 101 | 34 | 20.99 | 0.860 (0.519,1.424) |
| RB1 high (>311.3) | 102 | 28 | NE | 103 | 56 | 12.98 | 0.438 (0.277,0.692) |
| RB1 low (<=311.3) | 105 | 30 | NE | 100 | 39 | 20.99 | 0.718 (0.444,1.163) |
| TFDP1 high (>417.7) | 100 | 26 | NE | 105 | 56 | 12.98 | 0.420 (0.263,0.671) |
| TFDP1 low (<=417.7) | 107 | 32 | NE | 98 | 39 | 15.97 | 0.743 (0.464,1.190) |
| NE=not estimable | | | | | | | |
| Median (time to event) are generated by Kaplan-Meier survival method. | | | | | | | |
| Hazard Ratio for Ribociclib 600mg + letrozole 2.5mg versus Placebo + letrozole 2.5mg. | | | | | | | |

The results of the study show that particular patients that have a particular biomarker signature (i.e., have an increase in one or more of the biomarkers shown in Table 1) have an increased chance of progression free survival when treated with the combination of ribociclib and letrozole then letrozole alone.

### Example 3: Method description for generation of the gene signature

A Cox Proportional Hazards model was fitted with treatment, liver / lung metastasis which is the study stratification factor, effects of the 16 genes, including CCNA2, CCND1, CCND2, CCND3, CCNE1, CDK2, CDK4, CDK6, CDKN1A, CDKN2A, CDKN2B, CDKN2C, E2F1, E2F3, RB1, and TFDP1, and gene by treatment interactions. A backward model selection was performed to select fitted variables that are significantly associated with progression free survival (PFS). A set of genes (CDKN2A and E2F3) with significant gene by treatment interactions in the model were identified through backward model selection. Signature scores of all samples were computed as the weighted sum of the gene expression values, where weights were the regression coefficients of respective gene by treatment interactions in the Cox Proportional Hazards model. Patients were classified into high and low subgroups using median signature score as the cut-off. Median PFS and hazard ratio together with 95% confidence intervals were computed for each patient subgroup.

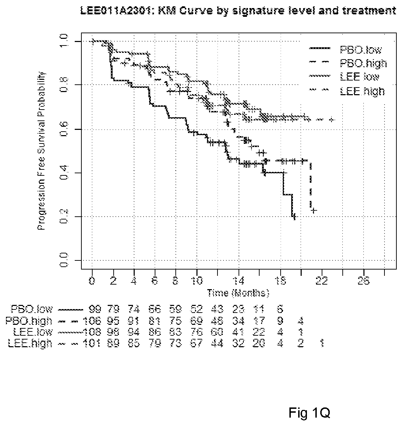

Result: Using backward model selection, a 2-gene signature (CDKN2A and E2F3) was derived. Figure 1Q and table 4 are subgroup analysis results, high- and low- gene signature expression groups are defined by median signature score as cut-off.

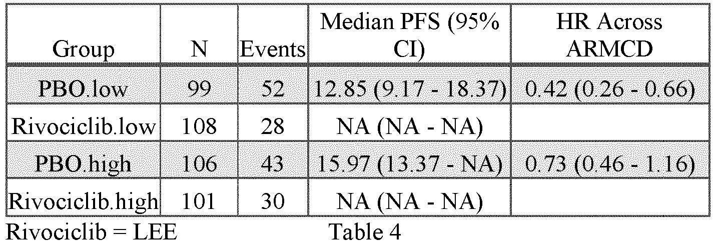

## Claims

1. A CDK 4/6 inhibitor which is ribociclib, or a pharmaceutically acceptable salt, solvate, hydrate or cocrystal thereof, for use in a method of selectively treating a patient having breast cancer, comprising:
a) assaying a biological sample from the patient for the level of expression of one or more of CCNA2, CCNE1, CDK2, CDK4, CDKN1A, CDKN2C, E2F1, E2F3, and RB1; and thereafter, selectively administering a therapeutically effective amount of the CDK 4/6 inhibitor and an aromatase inhibitor selected from the group consisting of letrozole, anastrozole and exemestane to the patient if the patient has an increased level of expression or one or more of CCNA2, CCNE1, CDK2, CDK4, CDKN1A, CDKN2C, E2F1, E2F3, and RB1 relative to a control.

2. A method of predicting the likelihood that a patient having breast cancer will respond to treatment with a CDK 4/6 inhibitor which is ribociclib, or a pharmaceutically acceptable salt, solvate, hydrate or cocrystal thereof, and an aromatase inhibitor selected from the group consisting of letrozole, anastrozole and exemestane, comprising: assaying a biological sample from the patient for the level of expression of one or more of CCNA2, CCNE1, CDK2, CDK4, CDKN1A, CDKN2C, E2F1, E2F3, and RB1, wherein an increase in the level of expression of one or more of CCNA2, CCNE1, CDK2, CDK4, CDKN1A, CDKN2C, E2F1, E2F3, and RB1 relative to a control is indicative of an increased likelihood that the patient will respond to treatment with the CDK 4/6 inhibitor and the aromatase inhibitor.

3. A method according to claim 2, wherein the absence of an increase in the level of expression of one or more of CCNA2, CCNE1, CDK2, CDK4, CDKN1A, CDKN2C, E2F1, E2F3, and RB1 is indicative of a decreased likelihood that the patient will respond to treatment with the CDK 4/6 inhibitor and the aromatase inhibitor.

4. A CDK 4/6 inhibitor which is ribociclib, or a pharmaceutically acceptable salt, solvate, hydrate or cocrystal thereof, and an aromatase inhibitor selected from the group consisting of letrozole, anastrozole and exemestane for use in a method of treating a patient having breast cancer, comprising:
a) selecting the patient for treatment with a CDK 4/6 inhibitor and an aromatase inhibitor on the basis of the patient having an increase in the level of expression of one or more of CCNA2, CCNE1, CDK2, CDK4, CDKN1A, CDKN2C, E2F1, E2F3, and RB1 relative to a control; and
b) thereafter, administering a therapeutically effective amount of the CDK 4/6 inhibitor and the aromatase inhibitor to the patient.

5. A CDK 4/6 inhibitor and an aromatase inhibitor for use according to claim 4, wherein the method comprises an initial step of assaying a biological sample from the patient for the level of expression of one or more of CCNA2, CCNE1, CDK2, CDK4, CDKN1A, CDKN2C, E2F1, E2F3, and RB1

6. A CDK 4/6 inhibitor for use according to claim 1, or a CDK 4/6 inhibitor and an aromatase inhibitor for use according to claim 4 or 5, or a method according to claim 2, 3, or 5 wherein the biological sample is a tumor sample.

7. A CDK 4/6 inhibitor for use according to claim 1, or a CDK 4/6 inhibitor and an aromatase inhibitor for use according to claim 4-6, or a method according to claim 2, 3, or 6, wherein the step of assaying comprises a technique selected from the group consisting of Northern blot analysis, polymerase chain reaction (PCR), reverse transcription-polymerase chain reaction (RT-PCR), TaqMan-based assays.

8. A CDK 4/6 inhibitor for use according to claim 1, or a CDK 4/6 inhibitor and an aromatase inhibitor for use according to any one of claims 4-7, or a method according to claim 2, 3. 6 or 7, comprising determining the level of expression of at least two genes.

9. A CDK 4/6 inhibitor for use according to claim 1, or a CDK 4/6 inhibitor and an aromatase inhibitor for use according to any one of claims 4-8, or a method according to claim 2, 3, or 6-8 comprising determining the level of expression of at least five genes.

10. A CDK 4/6 inhibitor for use according to claim 1, or a CDK 4/6 inhibitor and an aromatase inhibitor for use according to any one of claims 4-9, or a method according to claim 2, 3, or 6-9, comprising determining the level of expression of at least eight genes.

11. A CDK 4/6 inhibitor for use according to claim 1, or a CDK 4/6 inhibitor and an aromatase inhibitor for use according to any one of claims 4-10, or a method according to claim 2, 3, 6-10, wherein the breast cancer is HR-Positive, HER2-Negative; HR-Positive, HER2-Negative, post-menopausal; or HR-Positive, HER2-Negative, pre-menopausal.

12. A CDK 4/6 inhibitor for use according to claim 1, or a CDK 4/6 inhibitor and an aromatase inhibitor for use according to any one of claims 4-11, or a method according to claim 2, 3, 6-11, wherein the aromatase inhibitor is letrozole.

13. Ribociclib in combination with letrozole for use in a method of treating a postmenopausal female patient with HR-positive, HER2- negative, advanced breast cancer, who has previously received no prior therapy for advanced breast cancer, comprising:
assaying a biological sample from the patient for the level of expression of one or more of CCNA2, CCNE1, CDK2, CDK4, CDKN1A, CDKN2C, E2F1, E2F3, and RB1; and
thereafter, selectively administering a therapeutically effective amount of ribociclib and letrozole to the patient if the patient has an increased level of expression or one or more of CCNA2, CCNE1, CDK2, CDK4, CDKN1A, CDKN2C, E2F1, E2F3, and RB1 relative to a control.

## Patentansprüche

1. CDK-4/6-Inhibitor, bei dem es sich um Ribociclib oder ein pharmazeutisch unbedenkliches Salz, Solvat, Hydrat bzw. einen pharmazeutisch unbedenklichen Cokristall davon handelt, zur Verwendung bei einem Verfahren zur selektiven Behandlung einer Patientin mit Brustkrebs, umfassend:
a) Testen einer biologischen Probe von der Patientin auf das Expressionsniveau eines oder mehrerer von CCNA2, CCNE1, CDK2, CDK4, CDKN1A, CDKN2C, E2F1, E2F3 und RB1; und danach selektives Verabreichen einer therapeutisch wirksamen Menge des CDK-4/6-Inhibitors und eines Aromatase-Inhibitors, der aus der Gruppe bestehend aus Letrozol, Anastrozol und Exemestan ausgewählt ist, an die Patientin, falls die Patientin ein erhöhtes Expressionsniveau oder eines oder mehrere von CCNA2, CCNE1, CDK2, CDK4, CDKN1A, CDKN2C, E2F1, E2F3 und RB1 relativ zu einer Kontrolle aufweist.

2. Verfahren zur Vorhersage der Wahrscheinlichkeit, dass eine Patientin mit Brustkrebs auf eine Behandlung mit einem CDK-4/6-Inhibitor, bei dem es sich um Ribociclib oder ein pharmazeutisch unbedenkliches Salz, Solvat, Hydrat bzw. einen pharmazeutisch unbedenklichen Cokristall davon handelt, und einem Aromatase-Inhibitor, der aus der Gruppe bestehend aus Letrozol, Anastrozol und Exemestan ausgewählt ist, anspricht, umfassend: Testen einer biologischen Probe von der Patientin auf das Expressionsniveau eines oder mehrerer von CCNA2, CCNE1, CDK2, CDK4, CDKN1A, CDKN2C, E2F1, E2F3 und RB1, wobei eine Erhöhung des Expressionsniveaus eines oder mehrerer von CCNA2, CCNE1, CDK2, CDK4, CDKN1A, CDKN2C, E2F1, E2F3 und RB1 im Vergleich zu einer Kontrolle eine erhöhte Wahrscheinlichkeit, dass die Patientin auf eine Behandlung mit dem CDK 4/6-Inhibitor und dem Aromatase-Inhibitor ansprechen wird, anzeigt.

3. Verfahren nach Anspruch 2, wobei das Fehlen einer Erhöhung des Expressionsniveaus eines oder mehrerer von CCNA2, CCNE1, CDK2, CDK4, CDKN1A, CDKN2C, E2F1, E2F3 und RB1 eine verminderte Wahrscheinlichkeit, dass die Patientin auf eine Behandlung mit dem CDK-4/6-Inhibitor und dem Aromatase-Inhibitor ansprechen wird, anzeigt.

4. CDK-4/6-Inhibitor, bei dem es sich um Ribociclib oder ein pharmazeutisch unbedenkliches Salz, Solvat, Hydrat bzw. einen pharmazeutisch unbedenklichen Cokristall davon handelt, und Aromatase-Inhibitor, ausgewählt aus der Gruppe bestehend aus Letrozol, Anastrozol und Exemestan, zur Verwendung bei einem Verfahren zur Behandlung einer Patientin mit Brustkrebs, umfassend:
a) Auswählen der Patientin für eine Behandlung mit einem CDK-4/6-Inhibitor und einem Aromatase-Inhibitor auf der Grundlage, dass bei der Patientin ein erhöhtes Expressionsniveau eines oder mehrerer von CCNA2, CCNE1, CDK2, CDK4, CDKN1A, CDKN2C, E2F1, E2F3 und RB1 relativ zu einer Kontrolle vorliegt; und
b) danach Verabreichen einer therapeutisch wirksamen Menge des CDK-4/6-Inhibitors und des Aromatase-Inhibitors an die Patientin.

5. CDK-4/6-Inhibitor und Aromatase-Inhibitor zur Verwendung nach Anspruch 4, wobei das Verfahren einen ersten Schritt umfasst, bei dem eine biologische Probe von der Patientin auf das Expressionsniveau eines oder mehrerer von CCNA2, CCNE1, CDK2, CDK4, CDKN1A, CDKN2C, E2F1, E2F3 und RB1 getestet wird.

6. CDK-4/6-Inhibitor zur Verwendung nach Anspruch 1 oder CDK-4/6-Inhibitor und Aromatase-Inhibitor zur Verwendung nach Anspruch 4 oder 5 oder Verfahren nach Anspruch 2, 3 oder 5, wobei es sich bei der biologische Probe um eine Tumorprobe handelt.

7. CDK-4/6-Inhibitor zur Verwendung nach Anspruch 1 oder CDK-4/6-Inhibitor und Aromatase-Inhibitor zur Verwendung nach Anspruch 4-6 oder Verfahren nach Anspruch 2, 3 oder 6, wobei der Schritt des Testens eine Technik umfasst, die aus der Gruppe bestehend aus Northern-Blot-Analyse, Polymerase-Kettenreaktion (PCR), Reverse-Transkription-Polymerase-Kettenreaktion (RT-PCR), Assays auf TaqMan-Basis ausgewählt ist.

8. CDK-4/6-Inhibitor zur Verwendung nach Anspruch 1 oder CDK-4/6-Inhibitor und Aromatase-Inhibitor zur Verwendung nach einem der Ansprüche 4-7 oder Verfahren nach Anspruch 2, 3. 6 oder 7, umfassend Bestimmen des Expressionsniveaus von mindestens zwei Genen.

9. CDK-4/6-Inhibitor zur Verwendung nach Anspruch 1 oder CDK-4/6-Inhibitor und Aromatase-Inhibitor zur Verwendung nach einem der Ansprüche 4-8 oder Verfahren nach Anspruch 2, 3 oder 6-8, umfassend Bestimmen des Expressionsniveaus von mindestens fünf Genen.

10. CDK-4/6-Inhibitor zur Verwendung nach Anspruch 1 oder CDK-4/6-Inhibitor und Aromatase-Inhibitor zur Verwendung nach einem der Ansprüche 4-9 oder Verfahren nach Anspruch 2, 3 oder 6-9, umfassend Bestimmen des Expressionsniveaus von mindestens acht Genen.

11. CDK-4/6-Inhibitor zur Verwendung nach Anspruch 1 oder CDK-4/6-Inhibitor und Aromatase-Inhibitor zur Verwendung nach einem der Ansprüche 4-10 oder Verfahren nach Anspruch 2, 3, 6-10, wobei der Brustkrebs HR-positiv, HER2-negativ ist, HR-positiv, HER2-negativ, postmenopausal ist oder HR-positiv, HER2-negativ, prämenopausal ist.

12. CDK-4/6-Inhibitor zur Verwendung nach Anspruch 1 oder CDK-4/6-Inhibitor und Aromatase-Inhibitor zur Verwendung nach einem der Ansprüche 4-11 oder Verfahren nach Anspruch 2, 3, 6-11, wobei es sich bei dem Aromatase-Inhibitor um Letrozol handelt.

13. Ribociclib in Kombination mit Letrozol zur Verwendung bei einem Verfahren zur Behandlung einer postmenopausalen Patientin mit HR-positivem, HER2-negativem, fortgeschrittenem Brustkrebs, die nicht bereits eine frühere Therapie gegen fortgeschrittenen Brustkrebs erhalten hat, umfassend:
Testen einer biologischen Probe von der Patientin auf das Expressionsniveau eines oder mehrerer von CCNA2, CCNE1, CDK2, CDK4, CDKN1A, CDKN2C, E2F1, E2F3 und RB1; und
danach selektives Verabreichen einer therapeutisch wirksamen Menge von Ribociclib und Letrozol an die Patientin, falls die Patientin ein erhöhtes Expressionsniveau oder eines oder mehrere von CCNA2, CCNE1, CDK2, CDK4, CDKN1A, CDKN2C, E2F1, E2F3 und RB1 relativ zu einer Kontrolle aufweist.

## Revendications

1. Inhibiteur de CDK 4/6, qui est le ribociclib, ou un sel, solvate, hydrate ou cocristal pharmaceutiquement acceptable de celui-ci, destiné à être utilisé dans une méthode de traitement sélectif d'un patient atteint d'un cancer du sein, comprenant :
a) l'analyse d'un échantillon biologique provenant du patient pour déterminer le taux d'expression de l'un ou plusieurs parmi CCNA2, CCNE1, CDK2, CDK4, CDKN1A, CDKN2C, E2F1, E2F3 et RB1 ; puis l'administration sélective d'une quantité thérapeutiquement efficace de l'inhibiteur de CDK 4/6 et d'un inhibiteur d'aromatase choisi parmi le létrozole, l'anastrozole et l'exémestane au patient si le patient présente un taux d'expression accru ou l'un ou plusieurs parmi CCNA2, CCNE1, CDK2, CDK4, CDKN1A, CDKN2C, E2F1, E2F3 et RB1 par rapport à un témoin.

2. Méthode de prédiction de la probabilité qu'un patient atteint d'un cancer du sein réponde à un traitement par un inhibiteur de CDK 4/6 qui est le ribociclib, ou un sel, solvate, hydrate ou cocristal pharmaceutiquement acceptable de celui-ci, et un inhibiteur d'aromatase choisi dans le groupe constitué par le létrozole, l'anastrozole et l'exémestane, comprenant : l'analyse d'un échantillon biologique provenant du patient pour déterminer le taux d'expression de l'un ou plusieurs parmi CCNA2, CCNE1, CDK2, CDK4, CDKN1A, CDKN2C, E2F1, E2F3 et RB1, une augmentation du taux d'expression de l'un ou plusieurs parmi CCNA2, CCNE1, CDK2, CDK4, CDKN1A, CDKN2C, E2F1, E2F3 et RB1 par rapport à un témoin indiquant une probabilité accrue que le patient réponde au traitement par l'inhibiteur de CDK 4/6 et l'inhibiteur d'aromatase.

3. Méthode selon la revendication 2, dans lequel l'absence d'augmentation du taux d'expression de l'un ou plusieurs parmi CCNA2, CCNE1, CDK2, CDK4, CDKN1A, CDKN2C, E2F1, E2F3 et RB1 indique une probabilité réduite que le patient réponde au traitement par l'inhibiteur de CDK 4/6 et l'inhibiteur d'aromatase.

4. Inhibiteur de CDK 4/6, qui est le ribociclib, ou un sel, solvate, hydrate ou cocristal pharmaceutiquement acceptable de celui-ci, et inhibiteur d'aromatase choisi parmi le létrozole, l'anastrozole et l'exémestane, destinés à être utilisés dans une méthode de traitement d'un patient atteint d'un cancer du sein, comprenant :
a) la sélection du patient pour un traitement par un inhibiteur de CDK 4/6 et un inhibiteur d'aromatase sur la base du fait que le patient présente une augmentation du taux d'expression de l'un ou plusieurs parmi CCNA2, CCNE1, CDK2, CDK4, CDKN1A, CDKN2C, E2F1, E2F3 et RB1 par rapport à un témoin ; et
b) ensuite, l'administration d'une quantité thérapeutiquement efficace de l'inhibiteur de CDK 4/6 et de l'inhibiteur d'aromatase au patient.

5. Inhibiteur de CDK 4/6 et inhibiteur d'aromatase selon la revendication 4, la méthode comprenant une étape initiale d'analyse d'un échantillon biologique du patient pour déterminer le taux d'expression de l'un ou plusieurs parmi CCNA2, CCNE1, CDK2, CDK4, CDKN1A, CDKN2C, E2F1, E2F3 et RB1.

6. Inhibiteur de CDK 4/6 pour utilisation selon la revendication 1, ou inhibiteur de CDK 4/6 et inhibiteur d'aromatase selon la revendication 4 ou 5, ou méthode selon la revendication 2, 3 ou 5, l'échantillon biologique étant un échantillon de tumeur.

7. Inhibiteur de CDK 4/6 selon la revendication 1, ou inhibiteur de CDK 4/6 et inhibiteur d'aromatase selon les revendications 4 à 6, ou méthode selon la revendication 2, 3 ou 6, l'étape d'analyse comprenant une technique choisie dans le groupe constitué par l'analyse par Northern blot, la réaction en chaîne par polymérase (PCR), la réaction en chaîne par polymérase par transcription inverse (RT-PCR) et les analyses basées sur TaqMan.

8. Inhibiteur de CDK 4/6 selon la revendication 1, ou inhibiteur de CDK 4/6 et inhibiteur d'aromatase selon les revendications 4 à 7, ou méthode selon la revendication 2, 3, 6 ou 7, comprenant la détermination du taux d'expression d'au moins deux gènes.

9. Inhibiteur de CDK 4/6 selon la revendication 1, ou inhibiteur de CDK 4/6 et inhibiteur d'aromatase selon les revendications 4 à 8, ou méthode selon la revendication 2, 3, ou 6 à 8, comprenant la détermination du taux d'expression d'au moins cinq gènes.

10. Inhibiteur de CDK 4/6 selon la revendication 1, ou inhibiteur de CDK 4/6 et inhibiteur d'aromatase selon les revendications 4 à 9, ou méthode selon la revendication 2, 3, ou 6 à 9, comprenant la détermination du taux d'expression d'au moins huit gènes.

11. Inhibiteur de CDK 4/6 selon la revendication 1, ou inhibiteur de CDK 4/6 et inhibiteur d'aromatase selon les revendications 4 à 10, ou méthode selon la revendication 2, 3, ou 6 à 10, le cancer du sein étant HR-positif et HER2-négatif ; HR-positif, HER2-négatif et postménopausique ; ou HR-positif, HER2-négatif et préménopausique.

12. Inhibiteur de CDK 4/6 selon la revendication 1, ou inhibiteur de CDK 4/6 et inhibiteur d'aromatase selon les revendications 4 à 11, ou méthode selon la revendication 2, 3, ou 6 à 11, l'inhibiteur d'aromatase étant le létrozole.

13. Ribociclib en association avec le létrozole destinés à être utilisés dans une méthode de traitement d'une patiente ménopausée atteinte d'un cancer du sein HR-positif et HER2-négatif avancé, n'ayant reçu aucun traitement antérieur pour le cancer du sein avancé, comprenant :
l'analyse d'un échantillon biologique provenant du patient pour déterminer le taux d'expression de l'un ou plusieurs parmi CCNA2, CCNE1, CDK2, CDK4, CDKN1A, CDKN2C, E2F1, E2F3 et RB1 ; puis l'administration sélective d'une quantité thérapeutiquement efficace de ribociclib et de létrozole au patient si le patient présente un taux d'expression accru ou l'un ou plusieurs parmi CCNA2, CCNE1, CDK2, CDK4, CDKN1A, CDKN2C, E2F1, E2F3 et RB1 par rapport à un témoin.
